(19) Europäisches Patentamt
European Patent Office
Office européen des brevets

(11) EP 2 177 514 A1

(12) **EUROPEAN PATENT APPLICATION**
published in accordance with Art. 153(4) EPC

(43) Date of publication:
**21.04.2010 Bulletin 2010/16**

(21) Application number: **08790963.6**

(22) Date of filing: **08.07.2008**

(51) Int Cl.:
***C07D 301/06** (2006.01)*
***C07D 301/08** (2006.01)*
***C07B 61/00** (2006.01)*
***B01J 29/89** (2006.01)*
***C07D 303/04** (2006.01)*

(86) International application number:
**PCT/JP2008/062326**

(87) International publication number:
**WO 2009/008423 (15.01.2009 Gazette 2009/03)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.07.2007 JP 2007181973**

(71) Applicant: **Sumitomo Chemical Company, Limited**
**Tokyo 104-8260 (JP)**

(72) Inventors:
- **NARAHARA, Hideo**
  **Chiba 290-0142 (JP)**
- **HATANO, Ryo**
  **Ibaraki-shi**
  **Osaka 567-0841 (JP)**
- **KANAZAWA, Hideo**
  **Toyonaka-shi**
  **Osaka 561-0802 (JP)**

(74) Representative: **Vossius & Partner**
**Siebertstraße 4**
**81675 München (DE)**

# (54) APPARATUS AND METHOD FOR PRODUCING EPOXY COMPOUND

(57) The present invention provides an apparatus for producing an epoxy compound derived from an olefin by causing a reaction of hydrogen, oxygen, and the olefin in a liquid solvent in the presence of a catalyst in a reactor, wherein a vapor phase in the reactor contains an inert gas; hydrogen, an oxygen-containing gas with an oxygen concentration of at least 90% by volume, and the olefin are supplied to the vapor phase and/or liquid phase of the reactor to cause a reaction; and there exist a means for taking out the liquid phase containing the reaction product from the reactor.

[Fig. 1]

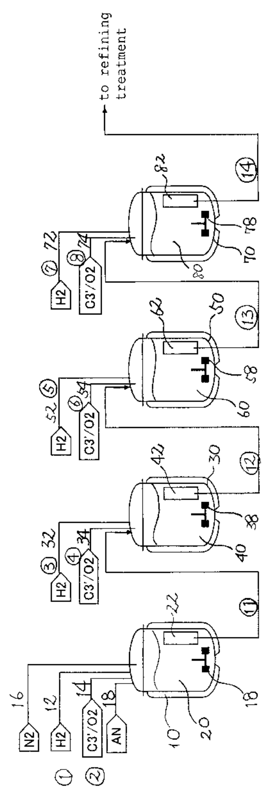


EP 2 177 514 A1

## Description

Field of the invention

The present invention relates to an apparatus and a method for producing an epoxy compound corresponding to an olefin by causing reaction of oxygen, the olefin, and hydrogen.

Description of the Related Art

Various methods of producing an epoxy compound corresponding to an olefin by causing reaction of the olefin such as propylene, hydrogen, and oxygen have been known (see Japanese Patent Kohyo Publication No. 2003-510314 and Japanese Patent Kohyo Publication No. 2005-514364 described later). In such methods, oxygen and hydrogen are used for epoxidation of an olefin to produce hydrogen peroxide. With respect to gases containing oxygen gas and hydrogen gas, the explosion range is very wide and direct mixing of them is very dangerous. Accordingly, in terms of the safety, as a mixture gas containing oxygen, an olefin, and hydrogen to be used for epoxidation reaction, oxygen diluted with nitrogen gas, an inert gas, is used and hydrogen and propylene are mixed with the diluted oxygen to produce the mixture gas.

SUMMARY OF THE INVENTION

[Problems to be solved by the Invention]

In the case of epoxidating propylene by diluting with nitrogen as described above, a mixture gas containing nitrogen gas, oxygen, propylene, and hydrogen is supplied to a reactor. The nitrogen gas contained in the mixture gas is substantially irrelevant to the reaction and flows together with the reaction product out of the reaction system and is finally discharged as a waste gas. Such a waste gas contains nitrogen gas as a main component and valuable components (e.g. propylene, propylene oxide, and the like) together with the nitrogen gas. Accordingly, in terms of economy and also in terms of environmental preservation, it is required to recover the valuable components.

Therefore, at the time of producing an epoxy compound corresponding to an olefin using the olefin, hydrogen, and oxygen, it is desired to provide a new production apparatus and production method for safely carrying out epoxidation reaction in place of a conventional method using nitrogen gas as a diluent gas.

[Means for solving the Problems]

The present inventors have made extensive studies on the above-mentioned problems and accordingly have found the matters as follows:

> that is, when an epoxy compound is produced by continuously supplying a olefin for the epoxy compound, hydrogen and oxygen without dilution with nitrogen gas to a reactor in the presence of a catalyst and they are reacted with one another in a liquid solvent, one can realize at least one of problems relating to a waste gas which is generated due to use of nitrogen gas as a diluent gas, preferably substantially can solve the problem, if the liquid phase containing the reaction product produced by the reaction is continuously taken out from the reactor.

Accordingly, in a first aspect, the present invention provides an apparatus for producing an epoxy compound derived from an olefin by causing a reaction of hydrogen, oxygen, and the olefin in a liquid solvent in the presence of a catalyst in a reactor, wherein the apparatus has a means for continuously supplying an inert gas to the vapor phase and/or liquid phase of the reactor, and a means for taking out the liquid phase containing the reaction product from the reactor, and wherein the reactor has a means for supplying hydrogen, an oxygen-containing gas with an oxygen concentration of at least 90% by volume and the olefin to the vapor phase and/or liquid phase in the reactor.
In other words, the present invention provides an apparatus for producing an epoxy compound derived from an olefin by causing a reaction of hydrogen, oxygen, and the olefin in a liquid solvent in the presence of a catalyst in a reactor, wherein a vapor phase in the reactor contains an inert gas; hydrogen, an oxygen-containing gas with an oxygen concentration of at least 90% by volume, and the olefin are supplied to the vapor phase and/or liquid phase of the reactor to cause a reaction; and there exist a means for taking out the liquid phase containing the reaction product from the reactor. The means for taking out the liquid phase from the reactor may be any proper means if it can take out the liquid phase from the reactor and for example, the means may be a pipe connected to the reactor and the pipe may be equipped properly with a pump, a valve, or the like.

Further, in a second aspect, the present invention provides a method for producing an epoxy compound derived

from an olefin by causing reaction of hydrogen, oxygen and the olefin in a liquid solvent in the presence of a catalyst in a reactor, **characterized in that** the process comprises a step of continuously supplying hydrogen, an oxygen-containing gas with an oxygen concentration of at least 90% by volume and the olefin to the vapor phase and/or liquid phase of the reactor, a step of reacting hydrogen, oxygen and the olefin in the liquid phase, and a step of taking out the liquid phase containing the reaction product from the reactor. In other words, the present invention provides a method for producing an epoxy compound derived from an olefin by causing reaction of hydrogen, oxygen and the olefin in a liquid solvent in the presence of a catalyst in a reactor, wherein at the time of supplying hydrogen, oxygen and the olefin to the reactor having a liquid phase containing liquid solvent and the catalyst and a vapor phase containing an inert gas to cause a reaction of them, the method comprising supplying hydrogen, an oxygen-containing gas with an oxygen concentration of at least 90% by volume and the olefin to the vapor phase and/or liquid phase of the reactor, reacting hydrogen, oxygen and the olefin in the liquid phase, and taking out the liquid phase containing the reaction product from the reactor. This production method is suitable for continuously producing an epoxy compound.

The present invention provides a production method including taking out a liquid phase containing a reaction product from a reactor. In this production method, it is preferable in terms of the safety to adjust the oxygen concentration in the vapor phase in the reactor outside of the explosion range thereof, preferably 5% or less. To solve the above-mentioned problems by decreasing an amount of a waste gas discharged from the reactor, in the case where hydrogen to be supplied to the reactor contains an inert gas other than hydrogen, and an olefin to be supplied to the reactor contains an inert gas other than hydrogen, and an oxygen-containing gas to be supplied to the reactor contains less than 10% by volume of an inert gas, the total amount of the inert gas, which inert gas being supplied to the reactor by supplying hydrogen, the oxygen-containing gas, and the olefin is preferably 10% by volume or less in the total amount of hydrogen, the oxygen-containing gas and the olefin.

[Effect of the Invention]

Both the production apparatus and production method of the epoxy compound according to the present invention mainly include that hydrogen, an oxygen-containing gas with an oxygen concentration of at least 90% by volume, and an olefin are reacted and a liquid phase containing the reaction product is taken out from a reactor as the main feature. According to the present invention, it is made possible to remarkably decrease the amount of the waste gas containing inert gas that is generated in a method using nitrogen gas as a diluent gas and thus at least one of the problems relevant to the waste gas can be moderated.

BRIEF DESCRIPTION OF THE DRAWINGS

Fig. 1 is a flow sheet schematically showing one embodiment of a production apparatus of an epoxidation according to the present invention;
Fig. 2 is a flow sheet schematically showing another embodiment of a production apparatus of an epoxidation according to the present invention;
Fig. 3 is a flow sheet schematically showing further another embodiment of a production apparatus of an epoxidation according to the present invention;
Fig. 4 is a flow sheet schematically showing further another embodiment of a production apparatus of an epoxidation according to the present invention;
Fig. 5 is a flow sheet schematically showing further another embodiment of a production apparatus of an epoxidation according to the present invention;
Fig. 6 is a flow sheet showing one example of the process for refining treatment of the liquid phase of a reactor containing an epoxy compound as a reaction product;
Fig. 7 is Table 3 showing the balance of substances in Example 1;
Fig. 8 is a flow sheet of a reaction apparatus of Comparative Example 1;
Fig. 9 is Table 4 showing the balance of substances in Comparative Example 1;
Fig. 10 is Table 5 showing the calculation results of the vapor phases and partial pressures of the third reactor and reactors thereafter in the apparatus of Comparative Example;
Fig. 11 is Table 6 showing the residence time to obtain the same PO production velocity as that of Reference Example in Comparative Example 1.

EXPLANATION OF Reference Numbers

**[0011]**

| | |
|---|---|
| 10: | first reactor |
| 12: | hydrogen stream |
| 14: | oxygen-containing gas/olefin gas mixture |
| 16: | inert gas |
| 18: | stirrer |
| 20: | liquid phase |
| 22: | filtration apparatus |
| 30: | first reactor |
| 32: | hydrogen stream |
| 34: | oxygen-containing gas/olefin gas mixture |
| 36: | inert gas |
| 38: | stirrer |
| 40: | liquid phase |
| 42: | filtration apparatus |
| 50: | first reactor |
| 52: | hydrogen stream |
| 54: | oxygen-containing gas/olefin gas mixture |
| 56: | inert gas |
| 58: | stirrer |
| 60: | liquid phase |
| 62: | filtration apparatus |
| 70: | first reactor |
| 72: | hydrogen stream |
| 74: | oxygen-containing gas/olefin gas mixture |
| 76: | inert gas |
| 78: | stirrer |
| 80: | liquid phase |
| 82: | filtration apparatus |
| 200: | vapor phase |
| 202: | joined reactor |
| 204: | fan |
| 206, 208, 210, and 212: | pump |
| 214: | purge line |
| 300: | blower |
| 304: | vapor phase |
| 310: | purge line |
| 320: | recycle line |
| 400: | gas-liquid separator |
| 402: | vapor phase |
| 404: | liquid phase |
| 406: | blower |
| 410: | purge line |
| 420: | gas-liquid mixed phase stream |
| 500: | epoxidation production apparatus |
| 502: | first reactor |
| 504: | second reactor |
| 506: | third reactor |
| 508: | fourth reactor |
| 510: | gas-liquid separator |
| 512: | vapor phase |
| 514: | liquid phase |
| 518: | blower |
| 522: | purge line |

DETAILED DESCRIPTION OF THE PREFERRED EMBODIMENTS

Each of the production apparatus and production method of the epoxy compound according to the present invention has the feature that hydrogen, an oxygen-containing gas with an oxygen concentration of at least 90% by

volume, and an olefin are reacted and a liquid phase containing the reaction product is taken out from a reactor. Such invention will be described more in detail hereinafter, however such description is illustrative of any production apparatus and production method of epoxy compound according to the present invention, excluding the particular description only for the production apparatus and production.

The reactor to be used in the present invention is not particularly limited so long as it can be used for a reaction of hydrogen, an oxygen-containing gas, and an olefin in a liquid solvent in the presence of a catalyst to produce an epoxy compound. For example, a tank type reactor such as a stirring bath, a tower type reactor such as a bubbling tower may be used. In such a reactor, as described below, a liquid solvent and a catalyst are introduced therein and the reaction is caused.

In the present invention, the inert gas which is contained in the vapor phase in the reactor is inactive on the epoxidation reaction of hydrogen, an oxygen-containing gas and an olefin in a liquid solvent in the presence of a catalyst to produce an epoxy compound. That is, the term inert gas means a gas that substantially causes no adverse effect on the epoxidation reaction, which is exemplified by, for example, nitrogen, steam, carbon dioxide, argon, helium, and a mixture of any of these gases (e.g. a mixture of nitrogen and argon, a mixture of nitrogen and steam, and the like). Specifically, examples of the inert gas may include substances other than oxygen contained in the oxygen-containing gas (nitrogen, argon, and the like), impurities contained in hydrogen (carbon dioxide, methane, ethane, propane, and the like), impurities contained in the olefin (methane, ethane, propane, and the like).

Hydrogen, an oxygen-containing gas with an oxygen concentration of at least 90% by volume, and an olefin to be supplied to a reactor as reaction raw materials for producing an epoxy compound may be independently (that is, separately) supplied in one embodiment or a gas mixture of the oxygen-containing gas and the olefin, that is, an oxygen/olefin-containing gas may be supplied to the reactor and separately hydrogen may be supplied to the reactor in another embodiment. In yet another embodiment, a gas mixture containing hydrogen, the oxygen-containing gas, and the olefin, that is, oxygen/olefin/hydrogen-containing gas, may be supplied to the reactor. In this connection, these components to be supplied as reaction raw materials may be those which are obtained by separating and recovering substances including the epoxy compound produced in the production method according to the present invention and contained in the liquid phase and vapor phase taken out from the reactor or those which are recovered from other production processes under condition that they substantially cause no adverse effect on the method of the present invention. That is, impurities may be contained to an extent that they substantially cause no adverse effect on the method of the present invention.

As described above, in the case of supplying raw materials as a gas mixture, it is required to lower the possibility of explosion of the gas mixture as much as possible, since a mixture of oxygen and a combustible gas is to be supplied. Therefore, in the case of supplying them as a gas mixture, it is preferable to supply the gas mixture being diluted with the above-mentioned inert gas and accordingly, the reaction raw materials are preferable to be supplied as a mixture further containing an inert gas. Consequently, the inert gas (in the case of being contained in the reaction raw materials) composes a portion of the inert gas contained in the vapor phase of the reactor in the present invention.

In the present invention, as described above, in the case where a gas mixture of oxygen-containing gas and an olefin is to be supplied to a reactor, the production apparatus of the present invention is preferable to further include an apparatus for preparing an oxygen/olefin-containing gas and this preparation apparatus is preferable to include a first mixer for preparing an oxygen/steam/olefin-containing gas by mixing an oxygen/steam-containing gas containing oxygen and steam with an olefin, and a steam removal apparatus, e.g. a condenser, for preparing an oxygen/steam/olefin-containing gas with a decreased amount of steam, as an oxygen/olefin-containing gas, by removing steam from the oxygen/steam/olefin-containing gas by, for example, condensation.

In this case, in the production method of the present invention, it is preferable to previously prepare an oxygen/olefin-containing gas and this preparation is preferable to include a first mixing step of preparing an oxygen/steam/olefin-containing gas by mixing an oxygen/steam-containing gas containing oxygen and steam with an olefin as well as a steam removal step of preparing an oxygen/steam/olefin-containing gas with a decreased amount of steam, as an oxygen/olefin-containing gas, by removing steam from the oxygen/steam/olefin-containing gas by, for example, condensation.

In the present invention, as described above, in the case of supplying a gas mixture containing hydrogen, an oxygen-containing gas, and an olefin together to a reactor, the production apparatus of the present invention is preferable to further include an apparatus for preparing an oxygen/olefin/hydrogen-containing gas. Such preparation apparatus is preferable to include a first mixer for preparing an oxygen/steam/olefin-containing gas by mixing an oxygen/steam-containing gas containing oxygen and steam with an olefin; a steam removal apparatus, e.g. a condenser, for preparing an oxygen/steam/olefin-containing gas with a decreased amount of steam, as an oxygen/olefin-containing gas, by removing steam from the oxygen/steam/olefin-containing gas by, for example, condensation; as well as a second mixer for preparing an oxygen/steam/olefin/hydrogen-containing gas, as an oxygen/olefin/hydrogen-containing gas, by mixing the oxygen/steam/olefin-containing gas with a decreased amount of steam with hydrogen.

In this case, in the production method of the present invention, it is preferable to previously prepare an oxygen/olefin-containing gas and this preparation is preferable to include a first mixing step of preparing an oxygen/steam/olefin-containing gas by mixing an oxygen/steam-containing gas containing oxygen and steam with an olefin; a steam removal

step, e.g. a condensation step, of preparing an oxygen/steam/olefin-containing gas with a decreased amount of steam, as an oxygen/olefin-containing gas, by removing steam from the oxygen/steam/olefin-containing gas by, for example, condensation; as well as a second mixing step of preparing an oxygen/steam/olefin/hydrogen-containing gas, as an oxygen/olefin/hydrogen-containing gas, by mixing the oxygen/steam/olefin-containing gas with a decreased amount of steam with hydrogen.

As described above, if oxygen is diluted with steam, an oxygen/steam gas mixture with a low oxygen concentration can be obtained and therefore an olefin can safely be mixed therewith. Thereafter, an oxygen/olefin mixture gas containing a little amount of steam can be obtained after removing steam by, for example, condensation. Further, an oxygen/olefin/hydrogen-containing gas containing a little amount of steam can be obtained when hydrogen is added to the mixture gas. Such a gas is preferably usable in the production apparatus and production method of the epoxy compound according to the present invention. Particularly, in the case where water or a mixture of water and another organic compound (e.g. acetonitrile) is used as a liquid solvent, it is particularly preferable since the reaction system contains no unnecessary other components.

In the present invention, the oxygen-containing gas may be a gas containing substantially oxygen alone or a gas containing another gas in addition to oxygen. In the latter case, on the basis of the volume of the total oxygen-containing gas, the ratio of oxygen is preferable to be at least 90% by volume. It is because such an oxygen-containing gas can relatively easily and economically be made industrially available and the amount of the waste gas finally emanated outward from the reaction system can be suppressed. Another gas is an inert gas to the epoxidation reaction similar to the above-mentioned inert gas, and accordingly, the inert gas (in the case of being included in an oxygen-containing gas) as such another gas also composes a portion of the inert gas contained in the vapor phase in the reactor of the present invention. In the case where an inert gas contained in the oxygen-containing gas has the above-mentioned diluting function, reaction raw materials are not necessarily required to contain another inert gas for dilution.

The above-mentioned reaction raw materials to be supplied to the reactor may be supplied to the vapor phase, or to the liquid phase, or to the liquid phase and vapor phase existing in the reactor. In the case where the liquid phase and/or vapor phase is taken out from the reactor and externally circulated, the reaction raw materials may be supplied to the external circulation. When the reaction raw materials are supplied in such a manner, the reaction raw materials are dissolved in the liquid solvent, and then these reaction raw materials come to exist in the vapor phase depending on the composition of these reaction raw materials in the liquid solvent. In addition, the inert gas contained in the vapor phase, as described above, contains those derived from the oxygen-containing gas and/or from the reaction raw materials. When both of the oxygen-containing gas and the reaction raw materials contain no inert gas, the inert gas is separately supplied to the vapor phase of the reactor to make the vapor phase of the reactor contain the inert gas. In another embodiment, in addition to the inert gas derived from the oxygen-containing gas and/or the inert gas derived from the reaction raw materials, the inert gas may be separately supplied to the vapor phase of the reactor. In any case, to lower the amount of the waste gas being finally discharged out of the system finally, the amount of the inert gas to be supplied to the reactor is particularly preferably 10% by volume or less in the total of hydrogen, oxygen (that is, considering only oxygen contained in the oxygen-containing gas), and an olefin to be supplied to the reactor. In the case where the inert gas is not supplied to the reactor from the reaction raw materials and from the oxygen-containing gas, the inert gas may previously be supplied to the vapor phase to make the vapor phase of the reactor contain the inert gas. Thereafter, the inert gas is continuously or intermittently supplied to the vapor phase and/or liquid phase of the reactor in the case where the amount of the inert gas contained in the vapor phase is decreased. In a particularly preferable embodiment, the concentration of oxygen contained in the vapor phase of the reactor is 5% by volume or lower.

At the time of starting reaction, even if the kind of the inert gas contained previously in the vapor phase of the reactor is a different type from those of the inert gas contained in the supplied oxygen-containing gas and/or the inert gas diluting the reaction raw materials, as the raw material gases are continuously supplied and the reaction is continued, the composition of the inert gas contained in the vapor phase or the reactor becomes close to that of the inert gas contained in the oxygen-containing gas and the inert gas diluting the reaction raw materials and finally, becomes substantially same as them. Therefore, in one embodiment, at the time of starting the reaction, the type of the inert gas previously contained in the vapor phase of the reactor is not particularly limited and any inert gas may be contained. For example, in the case the oxygen-containing gas contains less than 10% by volume of argon and the reaction raw materials are diluted with nitrogen, the vapor phase of the reactor comes to finally contain nitrogen and argon regardless of the type of the inert gas existing there from the beginning.

In the present invention, the reaction raw materials are supplied to the vapor phase and/or liquid phase of the reactor, and even if the reaction raw materials are supplied to any phase, the vapor phase of the reactor contains hydrogen, oxygen, and the olefin as the reaction raw materials in addition to the above-mentioned inert gas and ideally, the vapor phase contains the reaction raw materials with a composition at equilibrium to the composition of the reaction raw materials contained in the liquid solvent. In this connection, it can easily be understood that the epoxy compound and other byproducts produced along with the proceeding of the reaction may be contained in the vapor phase of the reactor.

When the above-mentioned reaction raw materials are reacted in the liquid solvent in the reactor, an epoxy compound corresponding to the olefin as the reaction raw material is produced and is contained in the liquid solvent. In this present invention, the term "liquid phase containing reaction products" means such liquid solvent containing the epoxy compound. Accordingly, the "liquid phase containing reaction products" contains, in addition to the epoxy compound as the reaction product, the reaction raw materials dissolved in the liquid solvent (that is, oxygen, hydrogen, and the olefin), reaction byproducts inevitably produced during the reaction (in a negligible quantity), and the inert gas dissolved therein depending on the composition and the partial pressure of the inert gas existing in the vapor phase of the reactor. To take out of such a liquid phase may be carried out continuously or intermittently, if necessary. In this connection, such taking out procedure of the liquid phase does not include an embodiment comprising taking out the liquid phase as a reaction field existing in the reactor and recycling it to the same reaction field.

In the case "liquid phase containing the reaction products" is taken out from the reactor as described above, the amount of the inert gas contained in the liquid phase is only the dissolved portion and accordingly, the amount is very small as compared with that in the case where the vapor phase is directly taken out from the reactor. In other words, in the case "liquid phase containing the reaction products" is taken out from the reactor, the amount of the inert gas contained in the vapor phase in the reactor is not so much decreased. Accordingly, to continue the epoxidation reaction, it is sufficient to supply only oxygen, hydrogen, and the olefin actually involved in the reaction. Since the amount of the inert gas in the reactor decreases by the amount of the inert gas dissolved in the "liquid phase containing the reaction products", to continue the reaction in steady state, it is preferable to continuously or intermittently supply the inert gas in an amount corresponding to the decreased amount to the reactor. If there is not a particular problem to decrease the inert gas in the amount corresponding to the amount of the inert gas dissolved in the "liquid phase containing the reaction products" in the reactor to continue the reaction, the inert gas may not necessarily be supplied.

As described above, in the case where the inert gas contained in the vapor phase contains the gas derived form the oxygen-containing gas and/or the gas derived from the reaction raw materials, the inert gas existing in the vapor phase is increased in some cases. That is, even if the "liquid phase containing the reaction products" is taken out and the dissolved inert gas is discharged out from the system, the amount of the inert gas existing in the vapor phase is substantially increased. In such a case, a portion of the vapor phase of the reactor is preferable to be taken out of the system.

Accordingly, in one preferable embodiment of the present invention, only the "liquid phase containing the reaction products" is continuously taken out of the system from the reactor but no vapor phase is taken out from the reactor. However, in the case where the vapor phase is taken out and recycled to the same reactor again is contained in the embodiment of taking out no vapor phase. If necessary, the inert gas in an amount corresponding to the amount of the inert gas contained in the liquid phase to be taken out is supplied to the reactor. In the case where no inert gas is contained in the reaction raw materials and the oxygen-containing gas, this embodiment is preferable.

In another embodiment, the "liquid phase containing the reaction products" is continuously or intermittently taken out of the system from the reactor and at the same time a portion of the vapor phase is continuously or intermittently taken out from the reactor to prevent or suppress increase of the inert gas contained in the reactor. This embodiment is preferable in the case where the reaction raw materials and/or the oxygen-containing gas contains the inert gas and where increasing the amount of the inert gas in the reactor can not be prevented merely by taking out the "liquid phase containing the reaction products" from the system in the reactor.

As described above, the liquid phase taken out from the reactor may be subjected to refining treatment for recovering an epoxy compound or the like contained therein. The recovered epoxy compound is refined and used thereafter for an intended use and on the other hand, the recovered oxygen, hydrogen, inert gas, olefin, and the like can be re-circulated to the reactor and reused. In another embodiment, the liquid phase taken out from the reactor is supplied to another reactor to further continue epoxidation reaction. Further, since the vapor phase taken out from the reactor contains valuable substances such as oxygen, hydrogen, olefin, epoxy compound, and the like in accordance with the composition of the liquid phase in addition to the inert gas, they may be subjected to separation treatment to recover these components. The recovered oxygen, hydrogen, olefin, and the like may be re-circulated to the reactor and reused.

Accordingly, in one embodiment of the present invention, the production apparatus of the epoxy compound includes a plurality of reactors (e.g. reactors in number of N) connected in series and at least one of a plurality of reactors has the characteristics of the above-mentioned production apparatus of the present invention (that is, the vapor phase of the reactor contains an inert gas and the liquid phase containing the reaction products is taken out from the reactor). For example, four reactors are connected in series. In the uppermost reactor in the first stage (the first reactor), the vapor phase in the first reactor contains an inert gas. Also in the first reactor, hydrogen, an oxygen-containing gas with an oxygen concentration at least 90% by volume and an olefin are supplied to the vapor phase and/or liquid phase to cause the reaction. The liquid phase containing the reaction products is taken out from the first reactor via a means for taking out the liquid phase and is supplied to the reactor in the second stage (the second reactor).

Similarly to the first reactor, the second reactor may be a reactor of the production apparatus of the present invention. The liquid phase containing the reaction products is taken out from the second reactor and supplied to the a

next reactor in the third stage. The second reactor may not be the production apparatus of the present invention as mentioned in the above, but may be a conventionally known production apparatus of the epoxy compound. However, in a preferable embodiment, a plurality of reactors are all the above-mentioned production apparatus of the present invention. That is, with respect to each reactor, each vapor phase of each reactors contains an inert gas and the liquid phase containing the reaction products is taken out from each of the reactors. The liquid phase taken out is supplied to the subsequent reactor (accordingly, the reaction products are transported from upstream to downstream) and the liquid phase taken out from the reactor in the fourth most downstream stage (the fourth reactor) may be subjected to the refining treatment for separating the reaction products as described above.

Accordingly, the production apparatus of the present invention including a plurality of reactors comprises N reactors (N is an integer of 2 or higher) from the first stage to the Nth stage, which reactors are connected in series on the basis of the transportation direction of the liquid phase containing the reaction products, wherein at least one reactor among the reactors of the first stage to the (N-1) th stage (which is at least the Kth stage reactor (K is an integer higher than 1 and smaller than N)) has the construction that the liquid phase containing reaction products is taken out from the Kth reactor and supplied to its subsequent reactor (the (K + 1)th stage) and the vapor phase of at least one reactor (the Kth stage reactor) contains an inert gas and hydrogen, oxygen, and an olefin are supplied to the vapor phase and/or liquid phase of at least one reactor (the Kth stage reactor).

As being understood easily, the present invention provides a method for producing an epoxy compound using a plurality of reactors connected in series as described above, wherein the method includes taking out a liquid phase containing reaction products from at least one reactor among reactors of the first stage to the (N-1)th stage and supplying the liquid phase to the next reactor following the former reactor and the vapor phase of at least one reactor contains an inert gas and hydrogen, oxygen, and an olefin are supplied to the vapor phase and/or liquid phase of at least one reactor.

In the production apparatus and production method of the present invention described above, it is preferable to take out the liquid phase containing reaction products from the reactor of the Nth stage (the last reactor). In a particularly preferable embodiment, with respect to all of the reactors, it is preferable to take out the liquid phase from each reactor and to subject the liquid phase taken out from the reactor in the final stage to a refining treatment as described above. In addition, it is preferable to prepare a plurality series of such production apparatuses each having a plurality of reactors as described above and arrange them in parallel. In such production apparatuses, even if the operation of one series of reactors is required to stop, it is advantageous that another series of reactors can be operated and the production output of the epoxy compound can easily be increased.

In the case of using a plurality of reactors, it is preferable to supply at least one of an oxygen-containing gas, hydrogen and an olefin to one or more of the other reactors besides the first reactor. In the case where these raw material gases are supplied to only the first reactor, the concentration of the raw material gases in the subsequent reactors gradually decreases and therefore the reaction rate decreases. Accordingly, it is preferable to supply at least one of these raw material gases, preferably all of them, to one of the subsequent reactors, for example, all of the reactors to suppress decrease of the reaction rate, preferably to retain or increase the reaction rate. In such case, if hydrogen peroxide remains in the downstream reactor, particularly in the lowermost downstream stage (the last reactor) and it may cause an adverse effect, it is preferable that hydrogen and oxygen are not added to the last reactor.

In the case of using a plurality of reactors, it is preferable to supply at least one of an oxygen-containing gas, hydrogen, and an olefin to another reactor in addition to the first reactor. In the case where these raw material gases are supplied to only the first reactor, the concentrations of the raw material gases in successive reactors are gradually lowered and therefore the reaction rate is decreased. Accordingly, at least one of these raw material gases, preferably all of them, is supplied to one of the successive reactors, for example, all of the reactors to suppress decrease of the reaction rate, preferably to retain or increase the reaction rate. In this case, if hydrogen peroxide remains in the downstream reactor, particularly in the most downstream stage (the last reactor) and it may possibly cause an adverse effect, it is preferable that hydrogen and oxygen are not added to the last reactor.

The reactors to be used for the above-mentioned production apparatus and production method may be any proper reactor. Since the reaction progresses in the liquid solvent in the presence of a catalyst as described below and on the other hand, the reaction raw materials are supplied in a gas state to the reactor, the reactor which facilitates gas-liquid contact or mixing of gas with liquid is preferable. For example, the reactor is preferably a type for circulating the vapor phase and/or liquid phase. In one embodiment, the reactor is a type for externally circulating the liquid phase by a compressor and in another embodiment, the reactor is a type for externally circulating the liquid phase (vapor phase of a catalyst, bubbles, and the like may be contained) and the vapor phase by an ejector. In yet another embodiment, the reactor is a type for forcibly internally circulating the vapor phase to the liquid phase and particularly preferably includes so-called self-aspirating type gas-liquid contact stirrer. Further, the reactor may include a stirrer of a type entraining a portion of the vapor phase positioned on the top surface of the liquid phase. In yet another embodiment, the reactor may be a bubbling tower type. Particularly, a reactor equipped with an ejector is preferable and therefore, the liquid phase (which may contain a catalyst, bubbles, and the like) of the reactor can be externally circulated by the ejector and the vapor phase (existing adjacently to the surface of the liquid phase) can thus be suctioned by the ejector

based on the generated negative pressure to improve the gas-liquid contact and promote dissolution of the reaction raw materials contained in the vapor phase in the liquid phase.

In the present invention, it is preferable to use a powder or granular type catalyst in the epoxidation reaction. Accordingly, with respect to such a catalyst, the reactor is preferable to be fixed bed, suspended bed, or fluidized bed reactor. Further, the reactor is preferable to have a filter unit inside and/or outside thereof and it is preferable that the catalyst can be separated by leading the liquid phase to the filter at the time of taking out the liquid phase.

Next, the production apparatus and production method of the epoxy compound according to the present invention will be described more in detail with reference to the drawings. Fig. 1 is a flow sheet schematically showing one embodiment of a production apparatus of an epoxy compound according to the present invention. In the illustrated embodiment, four reactors (stirring tank type reactors) 10, 30, 50, and 70 of the present invention are connected in series on the basis of the liquid phase transportation direction. Nitrogen, as an inert gas, is previously supplied to the vapor phase of each reactor and a liquid solvent, as the liquid phase, (e.g. acetonitrile) is supplied to each reactor and a catalyst is retained therein in suspended state by the stirrers 18, 38, 58, and 78. Before starting the reaction, the vapor phase and liquid phase in each reactor are in the equilibrium state and nitrogen contained in the vapor phase is also dissolved in the liquid phase.

At the time of starting the reaction, hydrogen (H2 in Fig. 1) 12 and an olefin (C3', e.g. a mixture gas containing propylene and an oxygen-containing gas (02) (C3'/02 in Fig. 1)) 14 are supplied to the first reactor 10. In this embodiment, the oxygen-containing gas contains substantially only oxygen. Further, an inert gas (e.g. nitrogen, N2 in Fig. 1) 16 and a liquid solvent (e.g. acetonitrile, AN 18 in Fig. 1) are supplied to the reactor 10. Since this liquid solvent (e.g. acetonitrile) generally contains no inert gas (e.g. nitrogen), the inert gas (e.g. nitrogen) in the vapor phase in the reactor 10 comes to dissolve therein. In the first reactor 10, hydrogen, an oxygen-containing gas and an olefin are reacted in the liquid solvent under reaction conditions to produce an epoxy compound and the first reactor contains a liquid phase 20 containing such reaction products. The liquid phase is taken out from the first reactor 10 via a filter (inner filter) 22 and supplied to the subsequent second reactor 30 positioned in downstream. Since the liquid phase 20, as described above, contains oxygen, hydrogen, the olefin, its epoxy compound, and the inert gas (e. g. nitrogen) and is discharged out from the reaction system of the first reactor 10, hydrogen 12, oxygen and olefin 14 in an amount corresponding to the total of the amount consumed in the reaction and the amount being discharged are supplied to the first reactor 10 and also the inert gas (e.g. nitrogen) 16 is supplied to the first reactor 10.

To a subsequent second reactor 30 which previously contains a vapor phase containing the inert gas (e. g. nitrogen), hydrogen 32 is supplied and a mixture gas 34 containing an olefin and an oxygen-containing gas is supplied and they are reacted in a liquid phase 40 to produce an additional epoxy compound. The liquid phase 20 taken out from the reactor 10 already contains the dissolved inert gas (e.g. nitrogen) and therefore, the liquid phase of the second reactor 30 substantially dissolves no further inert gas (e.g. nitrogen). Accordingly, it is no need to supply the inert gas (e.g. nitrogen) to the second reactor 30. The second reactor 30 has substantially the same construction as the first reactor 10, except that there is no means for supplying the inert gas (e.g. nitrogen).

Both the third reactor 50 (hydrogen 52, a mixture gas (C3'/02) 54, a liquid phase 60, a filter 62) and the fourth reactor 70 (hydrogen 72, a mixture gas (C3'/02) 74, a liquid phase 80, a filter 82) have the same construction as the second reactor 30. However, since the liquid phase 80 being taken out from the fourth reactor 70 contains a prescribed amount of the produced epoxy compound, it is subjected to refining treatment to separate the epoxy compound and the epoxy compound with high purity can be obtained. As being understood easily, since the liquid phase 80 contains the dissolved inert gas (e.g. nitrogen), the inert gas (e.g. nitrogen) is discharged out from the system composed of the four reactors. Accordingly, nitrogen 16 in an amount corresponding to the amount of the inert gas (e.g. nitrogen) discharged out from the system is supplied to the first reactor 10.

As being understood easily, in the process shown in Fig. 1, the inert gas supplied to the first reactor 10 is discharged out from the system via the liquid phase 40 of the second reactor, the liquid phase 60 of the third reactor, and the liquid phase 80 of the fourth reactor. Since the amount of such inert gas (e.g. nitrogen) corresponds to the amount being able to dissolve in the liquid solvent (e.g. acetonitrile) at the maximum, the amount of the nitrogen 16 to be supplied is considerably little. Accordingly, in the illustrated embodiment, the problem due to a large amount of the inert gas can considerably be relieved. Additionally, in this embodiment, with respect to the operation pressure of the reactors, if the reactor is operated in a manner that the operation pressure lowers with descending the reactor in the series thereof, transportation means such as a pump for transporting the liquid phase to downstream side can be omitted.

Fig. 2 is a flow sheet schematically showing another embodiment of the production apparatus of an epoxy compound according to the present invention. In the illustrated embodiment, the four reactors connected in series as shown in Fig. 1 are connected in a manner that the liquid phases of neighboring reactors are set adj acently and are modified to form a connected reactor 202 to integrally connect the vapor phases of the four reactors to form one vapor phase 200 (that is, the vapor phases are held in common). In this embodiment, the same as the case of Fig. 1, the inert gas (e.g. nitrogen) 16 is supplied to the first reactor 10 in an amount corresponding to the amount of the inert gas (e.g. nitrogen) dissolved in the liquid phase of the fourth reactor 70. Additionally, hydrogen 12 and a mixture gas 14 containing

an oxygen-containing gas and an olefin are supplied to the common vapor phase 200. To equalize the gas composition in the vapor phase, a fan 204 is provided to the vapor phase 200 of the reactor. In the illustrated embodiment, each liquid phase is taken out from each reactor by pumps 206, 208, 210 and 212, respectively, and supplied to each subsequent reactor after passing each filter (external filters) 22, 42, 62 and 82. The liquid phase taken out from the reactor 70 in the final stage may be subjected to the refining treatment similarly to the embodiment of Fig. 1.

In the case where the oxygen-containing gas is a gas containing an inert gas other than oxygen (referred to a "second inert gas" for distinction from the above-mentioned inert gas) and the amount of the second inert gas derived from the oxygen-containing gas to be supplied to the reaction system is higher than the amount of the second inert gas dissolved in the liquid phase 80 taken out of the reaction system from the fourth reactor, the amount of the second inert gas existing in the vapor phase 200 of the reactor increases. Thus, the increased second inert gas is discharged out from the reaction system through a purge line 214. In the case where the amount of the second inert gas is higher, the supply of the above-mentioned inert gas (e.g. nitrogen) 16 may be omitted. Alternatively, in place of the inert gas (e.g. nitrogen) 16, the second inert gas may be supplied to the reactor 10. In Fig. 2, the same symbols are assigned to the elements having similar functions as those in Fig. 1.

Fig. 3 is a flow sheet schematically showing another embodiment of a production apparatus of an epoxy compound according to the present invention. In the illustrated embodiment, the four reactors are connected in series as shown in Fig. 1, wherein each vapor phase in each reactor is supplied to each subsequent reactor and the vapor phase of a reactor is to be supplied to the next reactor and the vapor phase 304 in the fourth reactor 70 in the final stage is recycled to the first reactor 10 through a compressor (or blower) 300 and a line 320. This embodiment, similar to the case of Fig. 1, corresponds to the case that the oxygen-containing gas to be supplied together with an olefin contained only oxygen. As described with reference to Fig. 2, in the case where the oxygen-containing gas contains the second inert gas, a portion of the vapor phase taken out from the fourth reactor 70 may be discharged out from the reaction system. In Fig. 3, the same symbols are assigned to the elements having the same functions as those in Fig. 1.

Fig. 4 is a flow sheet schematically showing another embodiment of a production apparatus of an epoxy compound according to the present invention. In the illustrated embodiment, the reactors are configured in a manner that the four reactors are connected in series similarly as shown in Fig. 1 and each vapor phase 21, 41, 61 and 81 is taken out from each reactor together with each liquid phase. This embodiment is useful in the case where it is impossible to discharge the entire amount of the inert gas being supplied to the reaction system outside of the reaction system only by the amount of the inert gas contained in the liquid phase of the fourth reaction. The gas-liquid mixed stream 420 taken out from the fourth reactor 70 is separated into the vapor phase 402 and the liquid phase 404 by a gas-liquid separator 400. The vapor phase 402 is re-circulated to the first reactor 10 through a compressor (or blower) 406. The liquid phase 404 may be subjected to the refining treatment similar to the embodiment described before. For the same purpose of the above-mentioned lines 214 and 310, a line 410 may be provided. Additionally, in Fig. 4, the same symbols are assigned to the elements having the same functions as those in Fig. 1.

Fig. 5 is a flow sheet schematically showing another embodiment of a production apparatus of an epoxy compound according to the present invention. In the illustrated embodiment, a fixed-bed type first reactor 502, a second reactor 504, a third reactor 506, and a fourth reactor 508 are connected in series. In this embodiment, similar to the case of Fig. 1, hydrogen 12, a mixture gas containing an oxygen-containing gas and an olefin 14 and an inert gas (e.g. nitrogen, N2 in Fig. 5) 16 are supplied to the first reactor 502 and only hydrogen and the mixture gas are supplied to each of the subsequent reactors. The stream discharged out from the final fourth reactor is separated into the vapor phase 512 and the liquid phase 514 by a gas-liquid separator 510. Similar to the embodiment of Fig. 4, the vapor phase 512 is recycled to the first reactor 502 via a compressor (or blower) 518 and the liquid phase 514 is subjected to the refining treatment.

In this embodiment, in the case where the first reactor to the fourth reactor (including the gas-liquid separator) are regarded as an integrated reactor500, the liquid phase 514 is taken out from the reactor 500 and on the other hand, the vapor phase containing the inert gas exists in a form of bubbles in the fixed-bed type reactor and at the same time, the vapor phase separated in the gas-liquid separator (that can be regarded as the vapor phase of the reactor500) 512 contains the inert gas. Additionally, in place of such a fixed-bed type reactor, a bubbling tower type reactor can be used similarly. In this embodiment, in the case where the oxygen-containing gas contains substantially only oxygen, the inert gas in the reaction system decreases in an amount corresponding to that dissolved in the liquid phase 514 being taken out. Thus, in order to supplement the inert gas in the amount corresponding to the decreased amount, the inert gas 16 is supplied from the outside of the system. On the other hand, in the case where the oxygen-containing gas contains the inert gas and the amount of the inert gas introduced into the system by the gas supply is more than the amount of the inert gas taken out from the system as that dissolved in the liquid phase 514, a portion of the vapor phase separated by the gas-liquid separator 510 is taken out from the system as a stream 522.

A production method of an epoxy compound for which the above-mentioned production apparatus and/or production method of the epoxy compound according to the present invention is particular preferably applied will be described in detail with reference to a case of using propylene as an olefin; however the production apparatus and/or production method of the epoxy compound according to the present invention can be applied for other conventionally

known reaction of producing an epoxy compound in a liquid solvent using oxygen, hydrogen, and an olefin in the presence of a catalyst.

The olefin particularly preferable to be used for the production apparatus and/or production method of the epoxy compound according to the present invention is propylene. Propylene to be used may be those which are produced by, for example, thermal cracking, catalytic decomposition of heavy oil, or catalytic reforming of methanol. Propylene may be refined propylene or crude propylene which is not particularly subjected to refining process unless any adverse effect is caused on the production apparatus and/or production method of the epoxy compound according to the present invention. Propylene to be used is generally propylene with purity of 90% by volume or higher and preferably propylene with 95% by volume or higher. Examples of such propylene may be those which contain, for example, propane, cyclopropane, methylacetylene, propadiene, butadiene, butanes, butenes, ethylene, ethane, methane, hydrogen and the like besides propylene.

In the production apparatus and/or production method of the epoxy compound according to the present invention, a gas mixture of an oxygen-containing gas and an olefin, for example, propylene with a composition out of an explosion range is previously prepared and it is supplied to a reactor to carry out epoxidation reaction. In the case a plurality of reactors are used, the reactors are preferably connected in series and a plurality of such series of the reactors are connected in parallel arrangement. Since a catalyst to be used in the epoxidation reaction shows high activity and high selectivity when being newly prepared and deactivated gradually along with proceeding of the reaction, use of a plurality of reactors makes it possible to separate a reactor containing the deactivated catalyst from the production line and separately regenerate the catalyst or load a new catalyst to the reactor and thus the operation can be carried out stably and continuously.

In the case a plurality of reactors are connected in series, a mixture gas containing an oxygen-containing gas and propylene as well as hydrogen may also be supplied to reactors following the first reactor, so that concentrations of oxygen, propylene and hydrogen in the second reactor and following reactors can be increased and the reaction rate can be improved. With respect to the additional propylene, propylene may be supplied to the respective reactors in form of a liquid, thereby the reaction velocity can be improved.

The insides of the reactors are filled with a titanosilicate catalyst and a palladium catalyst, which will be described later, and in the case of a fixed-bed type reactor, the catalysts are not continuously taken out or supplied. In the case a stirring bath for suspending the catalysts or a fluidized bed type reactor, a filtration apparatus is provided within the reactor and the catalysts previously loaded in the reactor are not continuously taken out and along with the activity deterioration of the catalysts, all or a part of the catalysts are discharged and regenerated in the outside of the reactor. In another embodiment, along with the activity deterioration of the catalysts, the catalysts are additionally loaded little by little and continuously or intermittently taken out in a form of a suspension together with the reaction liquid. In the case reactors in which the catalysts are suspended and which include no filtration apparatus in the inside are used and no filtration apparatus is provided with in the insides of the reactors but filtration is carried out between neighboring reactors in the outside of the reactors or at the outlet of the final reactor, the catalysts are continuously discharged out together with the liquid phase and filtered and thereafter, the filtered catalysts are turned back to the reactors.

Each reactor preferably includes a heat exchange function for controlling the temperature of the inside of the reactor and a mixing function for promoting and improving the contact among the catalysts, the gaseous reaction raw materials, with the liquid phase and dissolving the gases in the liquid phase as much as possible. Examples of reactors may be fixed-bed type reactors, fluidized bed type reactors, stirring tank type reactors, and outside circulation pumps and preferable examples are fixed-bed type reactors or stirring tank type reactors. For the purpose of improving the contact of gases with liquids, a diffusion plate and a sparger ring may be provided with a gas supply part; or in a stirring bath, a SCABA blade, a blade such as a basket type blade having a mechanism for diffusing bubbles into smaller ones, and a baffle plate may be provided. Gas may be supplied to the liquid phase or vapor phase. Alternatively, in the case a supplied gas reaches the vapor phase in a reactor before it is reacted, the un-reacted gas is likely to be lost through a gas discharge line. Thus, it is preferable to include a mechanism for re-dispersing the gas in the vapor phase to the liquid in order to avoid such a loss. A method for re-dispersing the gas in the liquid may be a method of providing a stirring blade having a hollow shaft; supplying a discharged liquid of an external circulation pump to a venturi to suction a gas in the vapor phase and discharged the gas in the liquid; or supplying the gas of the vapor phase to the liquid using a compressor or a blower.

In the case un-reacted hydrogen peroxide which is not consumed for the reaction remains in the reaction liquid, it may be possible that hydrogen peroxide is decomposed during post-treatment such as separation and refining thereby oxygen may be generated in an unexpected process, which may cause ignition and explosion. To prevent such phenomenon, neither oxygen-containing gas nor hydrogen is supplied to one or more reactors near the outlet among a plurality of reactors, thereby hydrogen peroxide which was produced in the prior reactors and not consumed in the reaction is reacted with propylene to eliminate partially or entirely.

In the case a reactor or a plurality of reactors are used, a liquid solvent is continuously supplied to the first reactor and examples of the liquid solvent may be water, organic solvents and a mixture of both. Examples of the organic

solvents may be alcohols, ketones, nitriles, ethers, aliphatic hydrocarbons, aromatic hydrocarbons, halohydrocarbons, esters, glycols and their mixtures. Examples of alcohols are methanol and 2-methyl-2-propanol. Examples of nitriles are C2-C4 alkylnitriles such as acetonitrile, propionitrile, isobutyronitrile, and butyronitrile and benzonitrile. In the case of using acetonitrile or a mixture of acetonitrile and water as the liquid solvent, the weight ratio of water and acetonitrile is generally in a range of (0 : 100) to (90 : 10) and preferably in a range of (0 : 100) to (50 : 50). If necessary, such a liquid solvent may be supplied to successive reactors, for example, the second reactor; however a liquid solvent, e.g. acetonitrile or a mixed solvent of acetonitrile and water, to be supplied to the reaction system is all supplied to the first reactor. The mixture gas of hydrogen, an oxygen-containing gas, and propylene is supplied to a reactor or to the first reactor and at least one of reactors following the first reactor in the case a plurality of reactors are used and consumed by reaction in the reactors. In this connection, it is preferable to omit the above-mentioned supply of the oxygen-containing gas and hydrogen, if necessary, in the reactor in the final stage or one or more reactors preceding the final reactor.

The oxygen-containing gas to be used for the mixture gas of the oxygen-containing gas and propylene contains 10% or less of an inert gas such as nitrogen, argon, and helium for preventing explosion in the total of oxygen and the inert gas (that is, oxygen concentration is 90% by volume or higher) and more preferably contains no inert gas (that is, substantially pure oxygen). In the case the mixture gas of the oxygen-containing gas and propylene contains a gas inactive to the reaction, the inert gas supplied to the reactor has to be discharged out of the reaction system and industrially, it is required to separate and recover propylene, propylene oxide, and the like, which are valuable components, contained in the waste gas. For example, in the case recovery is carried out by a facility such as a gas absorption tower, since a large amount of solvent is required and further since valuable components are recovered from a mixture of the solvent and the valuable components, it is disadvantageous in terms of energy and therefore, as the inert gas is less, it is more preferable. Alternatively, the waste gas is released as it is to the atmosphere or burned and detoxified and then released to the atmosphere and in this case, the valuable components are lost.

The ratio of the supply amount of acetonitrile or a mixed liquid of water and acetonitrile supplied as a liquid solvent to the propylene supply amount as a reaction raw material is generally in a range of 0.02 to 70 times, preferably 0.2 to 20 times, and more preferably 1 to 10 times as much as the total weight of them.

Acetonitrile may be crude acetonitrile produced as a byproduct in the acrylonitrile production process or refined acetonitrile. Refined acetonitrile with purity of generally 95% or higher, preferably 99% or higher, and more preferably 99.9% or higher can be used. Examples as crude acetonitrile may be those containing typically water, acetone, acrylonitrile, oxazole, allyl alcohol, propionitrile, hydrocyanic acid, ammonia, a trace of copper, iron or the like besides acetonitrile.

As the oxygen-containing gas, oxygen refined by cryogenic separation, oxygen refined by PSA (pressure swing adsorption) method, and oxygen contained in air can be used. However, in the case a large quantity of an inert gas such as nitrogen and argon is contained, the waste gas amount is increased and therefore, it is not preferable. In general, oxygen obtained by the PSA method has purity of 90% by volume or higher and use of the oxygen for the present invention is preferable since the waste gas amount is rather much reduced as compared with that in the case of using a gas mixture of air and propylene, however oxygen with purity of 99% by volume or higher can be used by the cryogenic separation method and the amount of the waste gas can further be reduced and it is more preferable.

As described, in this reaction, an oxygen-containing gas, hydrogen, and an olefin, for example, propylene are supplied to a reactor and reacted in the presence of a catalyst to produce an epoxy compound, for example, propylene oxide and in the case an oxygen-containing gas (optionally oxygen alone), hydrogen, and propylene are supplied simply to a reactor, the mixture may partially have a composition within an explosion range and there is a risk of occurrence of explosion. If the oxygen-containing gas (optionally oxygen alone) is diluted previously with whatever a gas to make the composition of oxygen-containing gas in out of the explosion range, the risk of the explosion could be extremely reduced even in the case it is mixed with any arbitrary gas. In the case a gas which is not reacted such as nitrogen, argon, and methane is used as a diluting gas for oxygen, the gas becomes a waste gas and therefore has to be discharged out of the system and requires post treatment as described above and therefore, use of the gas is not so much preferable.

In the case propylene alone is used as a diluent gas, it is not preferable since at the time of supplying, propylene can be utilized as a diluent gas for oxygen and in the case it reaches a reactor, it is consumed by being reacted with oxygen and therefore no waste gas is emitted. However, if an inert gas is additionally contained in the gas mixture of oxygen and propylene, the waste gas amount is decreased as compared with that in the case where merely an inert gas is used as a diluent gas for oxygen and therefore it is preferable. Excess propylene is dissolved in the liquid phase and transported in form of the liquid phase to the subsequent reactor and discharged out of the final reactor. The supply amount of oxygen is preferable to be adjusted at a ratio of propylene to oxygen (propylene/oxygen) of 45/55 or higher in terms of prevention of explosion of the mixture gas. In the case the ratio of propylene to oxygen is higher, the propylene amount to oxygen becomes excess and substantial amount of propylene remains unreacted. Then, such unreacted propylene is required to be recycled, which makes this case economically disadvantageous and it is important to keep the ratio of propylene to oxygen 95/5 or lower.

In the case it is required to carry out the reaction at a ratio of propylene to oxygen higher than 45/55 in terms

of the selectivity of the reaction and the conversion ratio, a gas mixture wherein the ratio of propylene to oxygen is adjusted higher than 45/55 may be supplied to the reactor or liquefied propylene may be supplied.

With respect to hydrogen, production method thereof is not particularly limited, however those produced by steam reforming of hydrocarbons can be used. Unless any adverse effect is caused on the epoxidation reaction (that is, the impurities are substantially inactive to the reaction, for example, the above-mentioned inert gases are contained as impurities), those having purity generally 80% by volume or higher, preferably 90% by volume or higher, and more preferably 99.9% or higher can be used. Similarly to the case of oxygen, it is not preferable that a large quantity of an excess inert gas is contained in hydrogen, since the waste gas amount is undesirably increased. The supply amount of hydrogen to supplying propylene is in a range generally 0.05 to 10 mol times, preferably 0.05 to 5 mole times. Similarly to the case of oxygen, in the case hydrogen contains a component which is not consumed in the reaction, the waste gas corresponding to that has to be discharged out of the system and it results in undesirable loss of the reaction raw materials and reaction auxiliary raw materials.

The volume ratio (oxygen/hydrogen) of oxygen to hydrogen in the vapor phase of a reactor is preferably 3.5 in the upper limit. Production of propane as a byproduct can be suppressed by adjusting the ratio to 3.5 or lower. The lower limit is generally 0.01 or higher and preferably 0.1 or higher.

The catalyst to be used for the epoxidation reaction of the present invention is a titanosilicate catalyst and a catalyst bearing noble metal (e. g. platinum, ruthenium, rhodium, iridium, osmium, gold and the like and preferably palladium).

The titanosilicate catalyst is not particularly limited if it is obtained by a portion of Si in a porous silicate is substituted with Ti and may include crystalline titanosilicates, layered titanosilicates, mesoporous titanosilicates. Examples of the crystalline titanosilicates may be for example, in accordance with the structure codes of IZA (international zeolite associate) TS-2 having MEL structure, Ti-ZSM-12 having MTW structure (described in Zeolites 15, 236-242 (1995)), Ti-Beta having BEA structure (described in Journal of Catalysis 199, 41-47 (2001)), Ti-MWW having MWW structure (described in Chemistry. Letters. 774-775 (2000)), Ti-UTD-1 having DON structure (described in Zeolites 15, 519-525 (1995)), TS-1 having MFI structure (described in Journal of Catalysis 130, 1-8 (1991)), and the like. Examples of the layered titanosilicates may be Ti-MWW precursor (described in Japanese Patent Application Kokai (JP-A) No. 2003-32745) and Ti-YNU (described in Angewante Chemie International Edition 43, 236-240 (2004)).

Examples of mesoporous titanosilicates may be Ti-MCM-41 (described in Microporous Material 10, 259-271 (1997)), Ti-MCM-48 (described in Chemical Communications 145-146 (1996)), Ti-SBA-15 (described in Chemistry of Materials 14, 1657-1664 (2002)), Ti-MMM-1 (described in Microporous and Mesoporous Materials 52, 11-18 (2002)), and the like. Crystalline titanosilicates or layered titanosilicates having fine pores with 12-membered or more oxygen rings are preferable. Examples of crystalline titanosilicates having fine pores with 12-member or more oxygen rings are Ti-ZSM-12, Ti-MWW, and Ti-UTD-1. Examples of layered titanosilicates having fine pores with 12-member or more oxygen rings are Ti-MWW precursor and Ti-YNU. More preferably, Ti-MWW and Ti-MWW precursor are used. The titanosilicate catalyst may be those obtained by silylation of silanol groups by silylation agents. Examples to be used as the silylation agent may be 1,1,1,3,3,3-hexamethyldisilazane, trimethylsilyl chloride, triethylsilyl chloride, and the like. The titanosilicate catalyst is generally treated with hydrogen peroxide water before use. The concentration of hydrogen peroxide water is in a range of 0.0001% by weight to 50% by weight.

A carrier of a catalyst bearing palladium on the carrier may be generally oxides such as silica, alumina, titania, zirconia, niobia, and the like; hydrates such as niobic acid, zirconic acid, tungstic acid, titanic acid, and the like; carbons such as activated carbon, carbon black, graphite, carbon nanotube, and the like; and titanosilicates. It is preferably carbon and more preferably activated carbon. Palladium can be deposited on a carrier by impregnating the carrier material in a palladium colloidal solution or in a solution wherein a palladium salt is dissolved. Examples of the palladium salt may be palladium chloride, palladium nitrate, palladium sulfate, palladium acetate, tetra-ammine palladium chloride, and the like. In the case of deposition using a colloid solution, it is generally preferable to calcine the impregnated carrier material under an inert gas atmosphere. In the case of deposition using a palladium salt, the salt is generally used after reduction of it with a reducing agent in liquid phase or vapor phase. In the case tetra-ammine palladium chloride is used, reduction may be carried out using ammonia generated by thermal decomposition in the presence of an inert gas after deposition.

The deposition amount of palladium is generally 0.01 to 20% by weight and preferably 0.1 to 5% by weight in the catalyst containing palladium deposited on the carrier. The catalyst containing palladium deposited on the carrier may further contain one or more kind noble metals other than palladium. Examples of noble metals other than palladium may be platinum, ruthenium, rhodium, iridium, osmium, and gold. The content of the noble metals other than palladium is not particularly limited.

The reaction type may be batch type, slurry-bed continuous circulation type, fixed-bed continuous circulation type, and the like and in terms of the productivity, the slurry-bed continuous circulation type and the fixed-bed continuous circulation type are preferable. In the case of the slurry-bed continuous circulation type, both of the titanosilicate catalyst and a catalyst containing palladium deposited on a carrier are filtered by a filter in the inside of a reactor or in the outside

and stagnated in a reactor. While a portion of the catalyst in a reactor is continuously or intermittently discharged out and regenerated and thereafter the regenerated catalyst may be turned back to the reactor, the reaction may be carried out, or while a portion is discharged outside of the system and a new titanosilicate catalyst and a new catalyst containing palladium deposited on a carrier in amounts corresponding to those discharged outside are added, the reaction may be carried out. The catalyst amount in a reactor is generally in a range of 0.01 to 20% by weight and preferably 0.1 to 10% by weight to the reaction solution.

In the case of the fixed-bed continuous circulation type, generally, the reaction and regeneration are reciprocally repeated to carry out reaction. At that time, it is preferable to use a catalyst obtained by forming using a excipient (filler) or a molding agent. The reaction temperature is in a range of generally 0 to 150°C, preferably 20 to 100°C, and more preferably 40°C to 70°C. The reaction pressure is in a range of generally 0.1 to 20 MPa and preferably 1 to 10 MPa.

In the epoxidation reaction of the present invention, it is preferable to produce an epoxy compound, for example, propylene oxide, at a high yield, it is preferable that one kind or a mixture of a plurality of quinoide compounds or one kind or a mixture of a plurality of ammonium compounds are subjected to the reaction, respectively alone or in combination. There are two type p-quinoide compound and o-quinoide compound as such quinoide compounds and the quinoide compounds to be used in the present invention include both of them.

As the quinoid compound, there are two types, i.e., a p-quinoid compound and a o-quinoid compound, both are included in the quinoid compound used in the present present invention.

Examples of the quinoid compound include a phenanthraquinone compound and a p-quinoid compound represented by the formula (1):

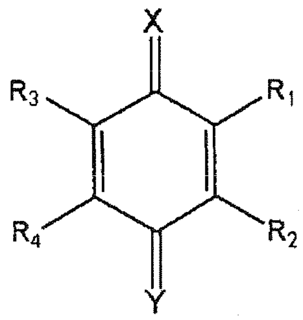

(1)

wherein $R_1$, $R_2$, $R_3$ and $R_4$ represent a hydrogen atom or, each adjacent $R_1$ and $R_2$, or $R_3$ and $R_4$ are independently bonded to each other at their terminal ends, and form a benzene ring optionally substituted with an alkyl group or a hydroxyl group, or a naphthalene ring optionally substituted with an alkyl group or a hydroxyl group together with the carbon atoms of the quinone to which they are bonded, and X and Y are the same or different and represent an oxygen atom or a NH group.

Examples of the compound represented by the formula (1) include

(1) a quinone compound (1A): the compound represented by the formula (1), wherein $R_1$, $R_2$, $R_3$ and $R_4$ are a hydrogen atom, and both X and Y are an oxygen atom;

(2) a quinone-imine compound (1B) : the compound represented by the formula (1), wherein $R_1$, $R_2$, $R_3$ and $R_4$ are a hydrogen atom, X is an oxygen atom, and Y is a NH group; and

(3) a quinone-diimine compound (1C): the compound represented by the formula (1), wherein $R_1$, $R_2$, $R_3$ and $R_4$ are a hydrogen atom, and both X and Y are a NH group.

The quinoid compound of the formula (1) includes a anthraquinone compound represented by the formula (2):

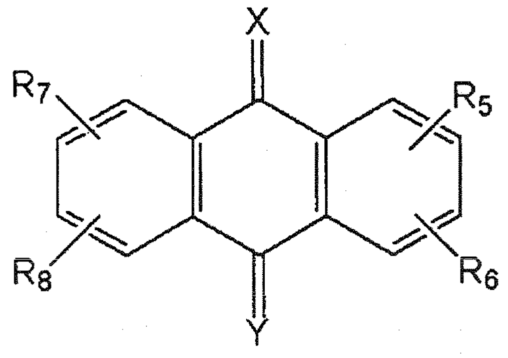

(2)

wherein X and Y are as defined in the formula (1), $R_5$, $R_6$, $R_7$ and $R_8$ are the same or different, and represent a hydrogen atom, a hydroxyl group, or an alkyl group (e.g., $C_1$-$C_5$ alkyl such as methyl, ethyl, propyl, butyl, pentyl, etc.).

In the formula (1) and formula (2), preferably, X and Y represent an oxygen atom. The quinoid compound represented by the formula (1) wherein X and Y are an oxygen atom is particularly referred to as quinone compound or p-quinone compound, and the quinoid compound represented by the formula (2) wherein X and Y are an oxygen atom is particularly referred to as anthraquinone compound.

Examples of the dihydro-form of the quinoid compound include dihydro-forms of the compounds represented by the foregoing formulas (1) and (2), i.e. compounds represented by the formulas (3) and (4):

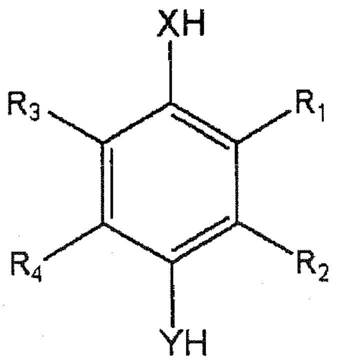

(3)

wherein $R_1$, $R_2$, $R_3$, $R_4$, X and Y are as defined in the foregoing formula (1); and

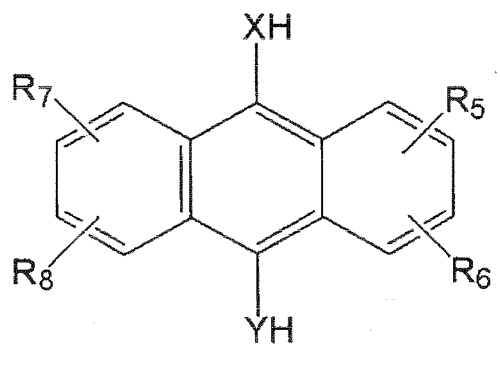

(4)

wherein X, Y, $R_5$, $R_6$, $R_7$ and $R_8$ are as defined in the foregoing formula (2).

In the formula (3) and formula (4), preferably, X and Y represent an oxygen atom. The dihydro-form of quinoid compound represented by the formula (3) wherein X and Y are an oxygen atom is particularly referred to as dihydroquinone compound or dihydro p-quinone compound, and the dihydro-form of quinoid compound represented by the formula (4)

wherein X and Y are an oxygen atom is particularly referred to as dihydroanthraquinone compound.

Examples of the phenanthraquinone compound include 1,4-phenanthraquinone as a p-quinoid compound and 1,2-, 3,4-, and 9,10-phenanthraquinone as o-quinoid compounds.

Specific examples of the quinone compound include benzoquinone, naphthoquinone, anthraquinone, 2-alkylanthraquinone compounds such as 2-ethylanthraquinone, 2-t-bytylanthraquinone, 2-amylanthraquinone, 2-methylanthraquinone, 2-butylanthraquinone, 2-t-amylanthraquinone, 2-isopropylanthraquinone, 2-s-butylanthraquinone and 2-s-amylanthraquinone, 2-hydroxyanthraquinone, polyalkylanthraquinone compounds such as 1,3-diethylanthraquinone, 2,3-dimethylanthraquinone, 1,4-dimethylanthraquinone and 2,7-dimethylanthraquinone, poylhydroxyanthraquinone such as 2,6-dihydroxyanthraquinone, naphthoquinone and a mixture thereof.
Preferred examples of the quinoid compound include anthraquinone, and 2-alkylanthraquinone compounds (in formula (2), X and Y are an oxygen atom, $R_5$ is an alkyl group substituted at 2 position, $R_6$ represents a hydrogen atom, and $R_7$ and $R_8$ represent a hydrogen atom). Preferred examples of the dihydro-form of quinoid compound include the corresponding dihydro-forms of these preferred quinoid compounds.

The addition of the quinoid compound or the dihydro-form of quinoid compound (hereinafter, abbreviated as the quinoid compound derivative) to a reaction solvent can be carried out by first dissolving the quinoid compound derivative in a liquid phase and then subjecting it to the reaction. For example, a hydride compound of the quinoid compound such as hydroquinone or 9,10-anthracenediol may be added to a liquid phase, followed by oxidation with oxygen in a reactor to generate the quinoid compound and use it in the reaction.

Further, the quinoid compounds used in the present present invention including the quinoid compounds exemplified above may become dihydro-forms of partly hydrogenated quinoid compounds depending on reaction conditions, and these compounds may also be used.

Such quinoide compounds may generally be supplied to the reactor in a dissolved form in acetonitrile. The lower limit of the supply amount is generally $1\times10^{-7}$ mole times and preferably $1\times10^{-6}$ mole times to supplied propylene. The upper limit depends on the solubility in a solvent and is generally 1 times by mole and preferably 0.1 times by mole.

In the method of the present invention, addition of an ammonium salt, that is, an ammonium, alkylammonium or alkylarylammonium salt, to the reaction solvent together with titanosilicate, a noble metal catalyst, and a quinoide compound is also effective to prevent the decrease of the catalyst activity, to increase the hydrogen utilization effect, and heighten the catalyst activity. The addition amount of an ammonium, alkylammonium or alkylarylammonium salt, is generally 0.001 mmol/kg to 100 mmol/kg per unit weight of solvent (total weight of a mixture of water and the organic solvent).

Such an ammonium salt may include salts made of 1) an anion selected from sulfate ion, hydrogen sulfate ion, carbonate ion, hydrogen carbonate ion, phosphate ion, hydrogen phosphate ion, dihydrogen phosphate ion, hydrogen pyrophosphate ion, pyrophosphate ion, halide ion, nitrate ion, hydroxide ion, or C1-C10 carboxylate ion and 2) a cation selected from ammonium, alkylammonium, and alkylarylammonium.

Examples of C1-C10 carboxylate ion are acetate ion, formate ion, acetate ion, propionate ion, butylate ion, valerate ion, caproate ion, caprylate ion, and caprate ion.

Examples of the alkyl ammonium include tetramethylammonium, tetraethylammonium, tetra-n-propylammonium, tetra-n-butylammonium and cetyltrimethylammonium.

Preferred examples of the salt selected from an ammonium salt, an alkyl ammonium salt or an alkyl aryl ammonium salt include ammonium salts of inorganic acids such as ammonium sulfate, ammonium hydrogen sulfate, ammonium carbonate, ammonium hydrogen carbonate, diammonium hydrogen phosphate, ammonium dihydrogen phosphate, ammonium phosphate, ammonium hydrogen pyrophosphate, ammonium pyrophosphate, ammonium chloride and ammonium nitrate; or ammonium salts of $C_1$ to $C_{10}$ carboxylic acids such as ammonium acetate, and a preferred ammonium salt is ammonium dihydrogen phosphate.

The ammonium salt is generally supplied to the reactor is a dissolved form in a solvent. The lower limit of the supply amount is generally $1\times10^{-6}$ mole times or higher and preferably $1\times10^{-5}$ mole times or higher to supplied propylene. The upper limit depends on the solubility in a solvent and is generally 2 mole times and preferably 0.2 mole times.

The refining treatment of the liquid phase containing an epoxy compound as a reaction product in a reactor or the final stage reactor obtained in the above-mentioned manner (hereinafter, referred to simply as "reaction solution") can be carried out by a process of which one example is shown in the flow sheet of Fig. 6. Since the reaction solution contains acetonitrile (including additive such as a quinoid compound, an ammonium, and the like), water, propylene oxide, propane, propylene, an inert gas, oxygen, hydrogen, and the like, these components are separated and recovered and used for a prescribed purpose (e.g. use as intended products, reuse by recycling to a reactor).

At first, after the reaction solution is flushed at a lower pressure than the operation pressure of a reactor, gas-liquid separation is carried out to obtain a gas mixture of such as an inert gas, oxygen, hydrogen and the like as low boiling point components and on the other hand, a liquid mixture containing acetonitrile (including additives such as a quinoide compound, ammonium and the like), water, propylene oxide, propane, propylene and the like. That is, the following A: gas-liquid separation process is carried out. Additionally, the above-mentioned gas mixture obtained by the

gas-liquid separation may be recycled together with the vapor phase taken out from a reactor to the epoxidation reaction process after organic components such as propylene oxide and propylene are removed by absorption process in B: waste gas refining process.

Next, for solvent recovery, the liquid mixture is distilled to separate a high boiling point component mixture containing acetonitrile and water as main components and a low boiling point component mixture containing propylene oxide, propane, propylene, water (in a little amount) and the like as main components. That is, the following C: solvent separation process is carried out. At that time, incondensable components produced at the time of obtaining the low boiling point component mixture may be treated by B: waste gas refining process.

After the low boiling point component mixture is dewatered through the dewatering treatment (after the following D: dewatering process is carried out), the low boiling point component mixture is subjected to distillation treatment to obtain crude propylene oxide mainly containing propylene oxide as a product and a low boiling point component mixture mainly containing propylene and propane (the following E: C3 separation process is carried out). At that time, incondensable components produced through the process obtaining the low boiling point component mixture may be treated by B: waste gas refining process. The low boiling point component mixture may be further distilled to separate propane and propylene and the propylene may be recycled to the epoxidation reaction process (that is, the following F: C3' recovery process is carried out).

Since the crude propylene oxide generally contain components with high polarity (byproducts of epoxidation reaction represented by acetaldehyde) and components with low polarity (byproducts of epoxidation reaction represented by alkanes, alkenes, and alkines having 4 to 6 carbon atoms), if necessary, it is subjected to refining process for removing them to obtain high purity propylene oxide (the following acetaldehyde separation process (not illustrated) and low polarity impurity separation process (not illustrated) are carried out).

A: Gas-liquid separation process The reaction solution can be separated into two phases; a vapor phase containing mainly un-reacted propylene, hydrogen and oxygen un-reacted or produced by decomposition of hydrogen peroxide, an inert gas, for example, nitrogen or the like and a liquid phase containing mainly propylene oxide, which is an intended product, and a solvent component; by lowering the pressure lower than that of the epoxidation reaction condition and these two phases can be successively separated by a proper method. The gas-liquid separation operation may be carried out by either batch process or continuous process. The gas-liquid separation operation may be carried out by one apparatus, a facility including two or more apparatuses arranged in series, or a facility including two or more apparatus rows each of which is formed by arranging one or more apparatuses in series. The gas-liquid separation operation may be carried out in a tank type manner, a tower type manner (in an empty tower and a packed tower), or the like. The separated liquid phase may be obtained as it is in a form of a liquid containing propylene oxide or refined through the following C: solvent separation process and successively following processes to obtain propylene oxide with high purity and at the same time separate the solvent or the like and the solvent may be recycled.

B: Waste gas refining process After treated by condensation operation, the vapor phase separated in A: gas-liquid separation process is treated by waste gas refining process, if necessary together with the vapor phase from C: solvent separation process and/or the vapor phase from E:C3 separation process. In this process, separation operation such as absorption, desorption, membrane separation using a proper solvent is carried out to recover propylene, hydrogen, and oxygen independently or together and recycle them for reaction.

C: Solvent separation process

The liquid phase separated in the gas-liquid separation process can be separated into a mixture containing mainly propylene oxide, propane, propylene, water, and the like and mixed components containing mainly solvent components by common operation such as distillation or adsorption. The separation operation may be carried out on the basis of difference of boiling points of object separation components or on the basis of difference adsorption properties. The separation operation may be carried out using a shelf tower, a bubble tower, a porous plate tower, a packed tower filled with a regular filler or an irregular filler, or the like and also a side cut operation may be employed. A distillation tower represented by Petluk-type distillation tower having a special structure in the tower can be employed.

A non-volatile inorganic weak basic or neutral salt is added to the liquid obtained by the gas-liquid separation process to be supplied to the distillation tower or adsorption tower, so that the propylene oxide can be prevented from conversion into propylene glycol. The non-volatile inorganic weak basic or neutral salt may be added previously to the object liquid to be treated by the distillation or adsorption operation or may be added separately to an arbitrary part of the distillation tower or adsorption tower.

To the liquid phase to be subjected to the solvent separation process, not only the liquid phase separated in the A: gas-liquid separation process but also at least a portion of the liquid phase obtained by condensing the vapor phase obtained in the gas-liquid separation process or at least a portion of a liquid phase obtained in another process having a proper composition may be added.

The vapor phase separated by the solvent separation process may be used as it is as a gas containing propylene oxide

or in the form of a liquid containing propylene oxide by condensing at least a portion of the gas or in the form of propylene oxide with high purity by refining in D: dewatering process described below and successively following processes.

D: Dehydration process

The mixture from which solvents are removed in the above-mentioned process still contains water. Since propylene oxide may possibly be converted into propylene glycol by the presence of the water and consequently the propylene oxide yield may be lowered or the property of the propylene oxide may be deteriorated, the water is preferable to be removed. A method for removing the water is not particularly limited to special methods and may be a method using an adsorbent, a method by membrane separation, and the like. In terms of the energy, a method by membrane separation is preferable. In the operation of removing the water, inevitable and simultaneous removal of other components is allowed if it occurs. In the water removal operation, even if a portion of propylene oxide is contained in the water to be removed is allowed, however in terms of improvement of the propylene oxide yield, it is better to be less. A method of removing the water is preferably a method of using a membrane and a method of chemical adsorption and physical adsorption using an adsorbent. The method of removing the water may be carried out using an apparatus for the process or, for example in pipes filled with adsorbents. The dewatering process may be carried out by batch operation or continuous operation. The method of removing the water may be carried out using one apparatus, a facility including two or more apparatuses arranged in series, or a facility including two or more rows of one or more apparatuses arranged in series. A portion or the full amount of an outlet solution of the apparatus for removing the water may be circulated to an inlet solution of the apparatus. The dewatering separation operation may be carried out in a tank type manner, a tower type manner, a porous plate tower, a packed tower type manner and the like.

E: C3 separation process

Since un-reacted propylene and propane are contained in the phase obtained by removing water from the reaction solution in the above-mentioned dewatering process, it is preferable to obtain crude propylene oxide containing mainly the propylene oxide by this separation operation. A method for the separation operation is not particularly limited to a specified method, however there is a method based on the difference of boiling points. Propylene and propane separated by the separation operation may contain a portion of components represented by propylene oxide. The phase containing the obtained propane and propylene may further be refined to separate it into a phase containing mainly propane and a phase containing mainly propylene and the phase containing mainly propylene may be a) recycled to the reaction; b) reformed to obtain Hydrogen that is recycled to the reaction; or c) used as fuel. The phase containing mainly propane may be a) used as a fuel or b) reformed by steam reforming to recover hydrogen that may be recycled to the reaction. In another embodiment, the phase containing mainly obtained propane and propylene may be used as a fuel for combustion. The phase containing propylene oxide separated by the separation operation as a main component may contain a portion of propylene and propane. The separation operation may be carried out by either batch operation or continuous operation, however from an industrial viewpoint, the continuous operation is preferable. The separation operation may be carried out using one apparatus, a facility including two or more apparatuses arranged in series, or a facility including two or more rows of one or more apparatuses arranges in series. The separation operation may be carried out in a tank type manner, a tower type manner, a porous plate tower, a packed tower type manner and the like.

F: C3' recovery process

The mixture containing propane and propylene obtained in the C3 separation process may be separated into propane and propylene by separation operation by distillation or the like. The separated propylene may be turned back to a reactor and used for reaction. On the other hand, the separated propane may be used as a fuel for combustion or used for steam reforming to obtain hydrogen that may be recycled to a reactor.

In the case it is required to remove the polar impurities and/or non-polar impurities contained in the crude propylene oxide obtained in the C3 separation process, the acetaldehyde removal process and/or low polarity impurity removal process, which will be described later, may be carried out.

Acetaldehydes removal process

The crude propylene oxide having proper purity can be obtained by the C3 separation process and since it contains a component having high polarity represented by acetaldehyde which is difficult to remove in the above-mentioned process, it is industrially desirable to remove it. In refining of propylene oxide, extraction and distillation methods are known well as a method for removing the component represented by acetaldehyde, and for example, acetaldehydes can be extracted and removed using acetonitrile. Commercialized acetonitrile or acetonitrile recovered

after extraction may be used for a portion or full amount of the acetonitrile or a solvent used in the epoxidation reaction and recovered in the above-mentioned C: solvent separation process may be used as it is with no treatment or after being refined. In the case propylene oxide is refined by the acetonitrile extraction process, since acetaldehydes are dissolved in acetonitrile, propylene oxide from which acetaldehydes are removed can be obtained. In the case acetonitrile is recovered as an extractant from a mixture of acetonitrile and acetaldehyde obtained after the extraction, a distillation method may be employed. In this distillation operation, acetonitrile or propylene oxide is supplied to distillation tower at or near the top of the tower, so that polymerization of acetaldehyde can be suppressed.

The extraction and separation operation may be carried out by either batch operation or continuous operation. The extraction and separation operation may be carried out using one apparatus, a facility including two or more apparatuses arranged in series, or a facility including two or more rows of one or more apparatuses arranges in series. The separation operation may be carried out in a tank type manner, a tower type manner, a porous plate tower, a packed tower type manner and the like. In the case the separation operation is carried out in a tank type manner or a packed tower type manner. Acetonitrile to be used as an extractant is preferable to be added at or near the top of the tower or at an inlet part of a heat exchanger for condensing a distilled fluid. In the case the separation operation is carried out in a tower type manner or a packed tower type manner, the raw material solution to be supplied to the operation is preferable to be supplied at the bottom part or near the bottom part of the tower. In the case the separation operation is carried out in a tower a porous plate tower manner or a packed tower type manner, propylene oxide from which acetaldehydes are removed is obtained from the top of the tower and a portion or full amount may be condensed and a portion or all condensed product can be re-circulated to the tower. The position for the return in the tower is not particularly limited; however it is preferable to be near the top of the tower. The propylene oxide obtained in the separation process may be used as a product as it is or further refined as described later. The components having high polarity and separated by the separation operation may be discarded as they are or distilled and separated or utilized advantageously after being extracted and separated with hydrocarbons having 6 to 12 carbon atoms.

Low polarity impurity removal process

Propylene oxide having proper purity can be obtained by the above-mentioned acetaldehyde removal process and the propylene oxide sometimes contain alkanes, alkenes, and alkines having 4 to 6 carbon atoms and difficult to be removed by the above-mentioned process and in such a case, it is preferable to remove them. As a method for removing alkanes, alkenes, and alkines having 4 to 6 carbon atoms, an extraction and distillation method using hydrocarbons having 6 to 12 carbon atoms as an extractant is known well and particularly preferably those having 7 or 8 carbon atoms are preferable. As the hydrocarbons having 6 to 12 carbon atoms to be used for the extraction and distillation method, commercialized ones may be used or hydrocarbons recovered after the extraction can be used for a portion or all of the extractant. In the case propylene oxide is refined by extraction operation using hydrocarbons having 6 to 12 carbon atoms, since the components having 4 to 6 carbon atoms are dissolved in the extractant having 6 to 12 carbon atoms, propylene oxide from which components having 4 to 6 carbon atoms are separated and removed can be obtained. The separation operation may be carried out by either batch operation or continuous operation.

The separation operation may be carried out using one apparatus, a facility including two or more apparatuses arranged in series, or a facility including two or more rows of one or more apparatuses arranges in series. The separation operation may be carried out in a tank type manner, a tower type manner, a porous plate tower, a packed tower type manner and the like. In the case the separation operation is carried out in a tower type porous plate tower manner, a porous plate tower, or a packed tower type manner, the components having 6 to 12 carbon atoms to be used as an extractant are preferable to be added at or near the top of the tower the top of the tower of the tower or an inlet part of a heat exchanger for condensing a distilled fluid. In the case the separation operation is carried out in a tank type manner or a packed tower type manner, the propylene oxide from which component having 4 to 6 carbon atoms are removed is obtained and a portion or the entire amount of it can be condensed and part or all of it can be re-circulated to the tower. The position for the return in the tower is not particularly limited; however it is preferable to be near the top of the tower. The propylene oxide obtained in the separation process may be used as a product as it is or further refined.

The refining treatment of the liquid phase containing an epoxy compound as a reaction product and taken out from the reactor or the final stage reactor in the present invention (hereinafter, referred to simply as "reaction solution") can be carried out as described above; however the above-mentioned refining treatment is merely an example and may be properly altered if necessary. For example, the liquid phase obtained in the gas-liquid separation process contains components such as the solvent, water, propylene oxide, propane, propylene and the like and they can be separated by the distillation process and the object components to be separated may be properly selected. For example, components with higher boiling points are successively separated and removed in this order or components with lower boiling points are separated in this order. Further, in another embodiment, a mixture containing higher boiling point components and a mixture containing lower boiling point components are separated and thereafter, intended components are separated and removed from these mixtures.

[Reference examples]

In the presence of a catalyst, propylene oxide was produced by reacting of oxygen, hydrogen, and propylene in water/acetonitrile as a liquid solvent. A titanosiliate and a palladium-bearing activated carbon were used as the catalyst.

The titanosilicate used was Ti-MWW having a titanium content of 1.8% by weight based on the ICP spectroscopy, which has been previously treated with hydrogen peroxide, which was prepared according to a method described in Chemistry Letters 774-775, (2000). That is, 0.133 g of a Ti-MWW powder was treated with about 80 cc of a solution of water/acetonitrile = 20/80 (weight ratio) containing 0.1% by weight of hydrogen peroxide for 1 hour at room temperature and washed with 100 ml of water and the obtained product was used for the reaction. As the noble metal catalyst, a 1 wt.% Pd/activated carbon produced as follows was used. That is, 300 mL of an aqueous solution containing 0.30 mmol of Pd tetraammine chloride was prepared in a 500 mL of an eggplant type flask. The aqueous solution was mixed with 3 g of a commercialized activated carbon powder (pore volume: 1. 57 cc/g, produced by Wako Pure Chemical Industries, Ltd.) and stirred for 8 hours. On completion of stirring, water was removed using a rotary evaporator and the activated carbon was impregnated with Pd tetraammine chloride. Further, vacuum drying was carried out at 50°C for 12 hours to obtain a catalyst precursor powder. The obtained catalyst precursor powder was calcined at 300°C for 6 hours in nitrogen atmosphere to obtain Pd/activated carbon catalyst.

The reaction was carried out using an autoclave having an inner cylinder of Teflon (trade name) with a capacity of 0.5 L as a reactor and a raw material gas of propylene/oxygen/hydrogen/nitrogen at 4.3/3.7/10.5/81.6 (by volume ratio) was fed at 20 L/hour and a solution of water/acetonitrile 20/80 (weight ratio) containing 0.7 mmol/kg of anthraquinone was supplied at 108 mL/hour and the liquid phase of the reaction mixture containing reaction products was taken out of the reactor via a filter to carry out a continuous reaction in conditions of temperature of 60°C, pressure of 0.8 MPa (gauge pressure), residence time 1.5 hours. During the reaction, 0.133 g of Ti-MWW and 0.03 g of Pd/activated carbon were made present in 131 g of the liquid solvent in the reactor. The liquid phase and vapor phase sampled at 5 hours after the start of the reaction were analyzed by gas chromatography and determined that the propylene oxide production activity per unit Ti-MWW weight was 35 mmol-PO/g-Ti-MWW.h. The selectivity on the basis of propylene was 90%, selectivity on the basis of hydrogen (produced propylene oxide mole/consumed hydrogen mole) was 46% and the remaining hydrogen peroxide was 0.38 mmol/hr.

Considering the production activity, selectivity and supply amount of the reaction raw material and the amount of the remaining hydrogen peroxide, and using the following elementary reactions for producing propylene oxide from oxygen, hydrogen, and propylene:

a*H2 + a*O2 → a*H2O2

b*H2O2 + b*H2 → 2b*H2O

c*H2O2 + c*C3' → c*PO + c*H2O

d*C3' + d*H2 → d*C3

(wherein C3': propylene, C3: propane, PO: propylene oxide): the coefficients a, b, c, and d could be estimated as

a = 0.007270

b = 0.002269

c = 0.004624

d = 0.000514.

The flow rates of the respective components contained in the stream flowing out of the reactor (that is, the totals of vapor phase and liquid phase of the respective components to be discharged) were estimated using the obtained coefficients. In this case, actually, the vapor phase and liquid phase were in the equilibrium state and the respective component flow rates of the vapor phase and liquid phase were calculated according to PSRK (Predictive Soave-Redlich Kwong-Gmehling) model to obtain the following results shown in Table 1.

Table 1

| | Flowing out stream | Vapor phase | Liquid phase |
|---|---|---|---|
| | mol/hr | mol/hr | mol/hr |
| C3' | 3.19E-02 | 2.65E-02 | 5.40E-03 |
| 02 | 2.46E-02 | 2.42E-02 | 4.66E-04 |
| H2 | 8.09E-02 | 7.99E-02 | 1.10E-03 |
| N2 | 7.07E-01 | 6.92E-01 | 1.45E-02 |
| PO | 4.62E-03 | 6.20E-04 | 4.00E-03 |
| AN | 1.72E+00 | 4.27E-02 | 1.68E+00 |
| H2O | 9.91E-01 | 1.83E-02 | 9.73E-01 |
| C3 | 5.14E-04 | 4.60E-04 | 5.37E-05 |
| Total | 3.56E+00 | 8.85E-01 | 2.68E+00 |

(AN: acetonitrile)

Since the hydrogen peroxide amount is a slight, it was ignored in the estimation.
The composition of the respective components was measured from the flow rates of the obtained vapor phase and the partial pressures of the respective components shown in the following Table 2 were calculated from the composition and the total pressure as the reaction condition.
Table 2

| | Vapor phase composition | Partial pressure |
|---|---|---|
| | mol% | kPa |
| C3' | 3.0 | 26.9 |
| 02 | 2.7 | 24.6 |
| H2 | 9.0 | 81.2 |
| N2 | 78.2 | 704.1 |
| PO | 0.1 | 0.6 |
| AN | 4.8 | 43.4 |
| H2O | 2.1 | 18.6 |
| C3 | 0.1 | 0.5 |
| Total | 100.0 | 900.0 |

The reaction was carried out by supplying raw materials in a manner that the residence time was adjusted to be 1.5 hours as described above and the partial pressure of hydrogen was maintained at 81 kPa to obtain 0.0046 mol/h of propylene oxide. That is, in the case of obtaining 0.0046 mol/h of propylene oxide under the experimental conditions, the ratio of residence time to hydrogen partial pressure (residence time/hydrogen partial pressure) was calculated and employed for the simulation of the following epoxidation reaction.

[Example 1]

The following matter was presumed at the time of simulation.
In the reaction of producing an epoxy compound using oxygen, hydrogen, and propylene, oxygen and hydrogen in the vapor phase were once dissolved in the liquid phase and the dissolved oxygen and hydrogen were converted to hydrogen peroxide by the palladium catalyst and the hydrogen peroxide in the liquid and propylene were epoxidated by the Ti-MWW catalyst to produce PO. The rate-controlling steps of the reaction were supposed to be (1) gas dissolution; (2)

hydrogen peroxide production in the liquid; and (3) PO production in the liquid and according to reaction experiments carried out variously by changing the conditions of the above-mentioned Reference Example, the reaction pressure and the PO production rate were found having mutually a positive correlation. That is, (1) was apparently the rate-controlling step.

On the other hand, the gas dissolution velocity J [mol/s/m3] can be expressed generally as the following equation.

$$J = kLa\ (C^* - C)$$

kLa [l/s] : substance moving capacity coefficient
C* [mol/m3] : saturated solubility of gas in liquid
C [mol/m3] : concentration of gas component in liquid

In this reaction, since the dissolution velocity J of the gas in the liquid became the rate-controlling step, the reaction velocity is r = J [mol/s/m3] and defined as the following equation.

$$r = kLa(C^* - C)$$

If it is assumed that the gas dissolution velocity is the rate-controlling step and the reaction velocities of (2) and (3) are faster than gas dissolution (1) and can be ignored, the gas concentration in the liquid becomes zero, that is, the reaction rate r can be defined as the following equation.

$$r = kLa \times C^*$$

From Henry's equation, the following relation can be established between the concentration C* of the gas in the liquid and gas partial pressure P.

$$C^* = P/k$$

k [Pa.m3/mol]: Henry's constant
Accordingly, the gas partial pressure P and the reaction velocity have the following relation.

$$r = kLa/k \times P$$

That is, the reaction velocity is proportional to the partial pressure. Hydrogen is considerably involves in the elementary reaction of the object reaction and thus it is preferable to evaluate the reaction velocity in consideration of the hydrogen partial pressure. In this connection, in Reference Example, the catalyst activity was 35 mmol-PO/g-TiMWW/hr when the H2 partial pressure was 81 kPa.

That is, the reaction velocity was proportional to the partial pressure. Hydrogen is considerably involves in the elementary reaction of the object reaction and thus it is preferable to evaluate the reaction velocity in consideration of the hydrogen partial pressure. In this connection, in Reference Example, when the H2 partial pressure was 81 kPa, the residence time of 1.5 hours is required to obtain 0.0046 mol/h of propylene oxide. On the other hand, the relation of the spatial time θ and the reaction velocity can be defined as the following equation according to the following Reference Document 1.

$$\theta = C0 \times x/(-r)$$

In the above-mentioned equation, x denotes reaction ratio [-], Co denotes reaction substrate concentration in

the supplied liquid [mol/m3], and r denotes the reaction rate [mol/m3/s]. According to the above-mentioned equation, it is understood that in the condition that the reaction ratio becomes constant, the spatial time θ is inversely proportional to the reaction velocity r and when r become two times, the spatial time becomes 1/2 times. As described, since the reaction velocity is inversely proportional to the gas partial pressure P, the gas partial pressure and the spatial time (that is, the residence time needed to obtain a prescribed reaction ratio) are inversely proportional to each other.
Reference Document 1: "reaction Engineering", HASHIMOTO Kenji, p. 54 Equation 3-45, Baifukan (5th edition, on Oct. 10, 1995).

Simulation of an epoxidation reaction facility of the present invention shown in Fig. 1 was carried out. The following assumptions were employed. The catalyst amounts in the inside of the reactor, temperature, and total pressure were same as Reference Example. Four reactors were completely mixing baths. Thus these reactions constitute (SCTR (continuous stirred tank reactor)). At first all of the baths were filled with 131 g of 80 wt.% aqueous acetonitrile water (i.e. acetonitrile/water mixed liquid) and previously pressurized with N2 and saturated.

The mixed liquid of acetonitrile and water at the same flow rate as that in Reference Example was continuously supplied. In the liquid phase of the flowing out stream in Reference Example, nitrogen dissolved in acetonitrile water was 1.45 E-02 mol/hr. Since nitrogen in this amount finally flowed out of the liquid phase of the fourth reactor, nitrogen in the same amount was supplied continuously to the first reactor.

The composition of the vapor phase in the first reactor was always kept constant by continuously supplying H2, 02, and C3' in amounts corresponding to the consumption to the first reactor in a manner that the composition became same as the above-mentioned composition of the vapor phase of the flowing out stream of the reactor in Reference Example and thus the same reaction velocity as that of Reference Example was obtained in the first reactor.

The liquid phase in an amount equivalent to the total amount supplied to the first reactor was continuously discharged out of the first reactor and supplied to the second reactor. In this Example, since the inert gas was supplied only in an amount corresponding to the amount dissolved in the liquid phase containing reaction products, the gas amount in the vapor phase of the first reactor was not changed and accordingly, no waste gas was generated and operation could be carried out in closed system (only the liquid phase was taken out and it was no need to discharge gas from the vapor phase).

In the vapor phase part of the second reactor same as that in the first reactor, the composition of the vapor phase was always kept constant by continuously supplying H2, 02, and C3' in amounts corresponding to the consumption to the first reactor and the same reaction velocity as that of the first reactor was obtained and no waste gas was generated in the second reactor.

The liquid phases were transported from the second reactor to the third reactor and also from the third reactor to the fourth reactor and as same in the second reactor, no waste gas was generated in the third reactor and the fourth reactor.

The substance supply and consumption (mass balance) of the respective streams are as shown in Table 3 of Fig. 7. The numbers of the streams in Table 3 correspond to the numbers marked with o (i.e. circled number) in Fig. 1. Nitrogen 16 and acetonitrile 18 as inert gases in Fig. 1 are shown as N2 and AN, respectively in Table 3. PO, C3, and water produced in the respective reactors are same as those shown Reference Example. The amounts of 02, H2, and C3' supplied to the first reactor are the amounts calculated by adding the amounts of them dissolved in the liquid and transported to the second reactor to the amounts consumed in Reference Example. The amounts of 02, H2, and C3' consumed in the second reactor or reactors thereafter are defined same as those in Reference Example and accordingly, the same amounts as those in Reference Example are added.

Since the vapor phase compositions of the respective reactors are adjusted to be same as those in Reference Example, the partial pressure of H2 becomes same as that in Reference Example and also in the first reactor, the second reactor, the third reactor, and the fourth reactor, the needed residence time is respectively same, 1.5 hours, and in total 6 hours. Further, the waste gas to be discharged out of the reaction system is zero.

[Comparative Example 1]

As shown in flow sheet of Fig. 8, four reactors were connected in series and the liquid phase and vapor phase of one reactor were made continuously transportable to the next reactor. The catalyst amounts in the reactors, temperature, and total pressure were same as those in Reference Example. Four reactors were completely mixing baths. At first all of the baths were filled with 131 g of 80 wt.% acetonitrile water and previously pressurized with N2 and saturated. Acetonitrile water and a gas mixture containing hydrogen, oxygen, nitrogen (the ratio of oxygen to oxygen and nitrogen was 4%), and propylene was continuously supplied to the first reactor. That is, PO, C3 and water in the completely same amounts of those in Reference Example were produced in the first reactor and H2, 02, and C3' were consumed and the vapor phase and liquid phase were both transported to the second reactor. The first reactor was completely same as that of Reference Example.

The gas supplied additionally to the second reactor had the same composition of the above-mentioned gas

mixture supplied to the first reactor and contained H2 in the amount corresponding to the H2 amount consumed in the first reactor.

The liquid phase and vapor phase of the first reactor were supplied to the second reactor. The catalyst concentrations in the second reactor were same as those in the first reactor. That is, the catalysts in the amounts were loaded in proportion to the liquid held up in the second reactor. The temperature and the total pressure in the second reactor were same as those in the first reactor.

Fresh reaction raw materials, H2, 02 and C3' were additionally loaded to the second reactor and since they were diluted by nitrogen contained in the vapor phase supplied from the first reactor, the partial pressures of the respective components were decreased than those in the vapor phase composition of the first reactor; however the liquid hold-up was set to be higher in the second reactor and the residence time was set long to make the production velocity of PO same as the first reactor.

Since the production velocity of PO in the second reactor was same as the first reactor and thus the velocities of H2, 02 and C3' consumed in the second reactor were same as the production velocity of the byproduct water and byproduct C3. Accordingly, the amount of the mixture as the total of the vapor phase and liquid phase discharged out of the second reactor (stream 16 in Fig. 8) was measured.

The residence time in the reactor 2 was calculated as follows. At first, the amount of the mixture (stream 16 in Fig. 8) as the total of the vapor phase and liquid phase discharged out of the second reactor, the respective components of the vapor phase and liquid phase supplied from the first reactor was calculated from the balance of the respective components to be consumed. Same as Reference Example, the vapor phase composition and the liquid phase composition were calculated using the PSRK model of the gas-liquid equilibrium composition. Since the total pressure was maintained as the same pressure as that in Example 1, the partial pressures of the respective components were calculated as products of the total pressure and the vapor phase composition.

As described in Reference Example, the residence time in the reactor required for producing the same PO as that of Reference Example was calculated.

The residence time was calculated in the same technique for the third reactor and reactors thereafter to obtain the results (the stream number in Table 4 (the number marked by o (i.e. circled number)) and a, b, and c are corresponding to the stream number and a, b, and c of Fig. 8) shown in Table 4 of Fig. 9. The vapor phase composition and the partial pressure of the third reactors and reactors thereafter were calculated to obtain results shown in Table 5 of Fig. 10. As described, the gas partial pressure and the spatial time (that is, the residence time needed to obtain a prescribed reaction ratio) are inversely proportional to each other, and accordingly, the residence time needed to obtain the same PO production velocity as that of Reference Example was calculated to find the result as shown in Table 6 of Fig. 11.

Although the production velocities of propylene oxide were same in Comparative Example 1 and Example 1, the waste gas amount in Example 1 was substantially zero, and on the other hand, a very large quantity of a waste gas was produced in Comparative Example 1. Further, in Comparative Example 1, the residence time in the second reactor and thereafter became long.

[Industrial Applicability]

An epoxy compound production apparatus and production method of the present invention are particularly useful for epoxidation of an olefin and particularly propylene.

In the present invention, preferred Embodiments are exemplified as follows:

(1) In Embodiment 1, in an apparatus for producing an epoxy compound derived from an olefin by causing a reaction of hydrogen, oxygen, and the olefin in a liquid solvent in the presence of a catalyst in a reactor, the apparatus has a means for continuously supplying an inert gas to the vapor phase and/or liquid phase of the reactor, and a means for taking out the liquid phase containing a reaction product from the reactor, and wherein the reactor has a means for supplying hydrogen, an oxygen-containing gas with an oxygen concentration of at least 90% by volume and the olefin to the vapor phase and/or liquid phase in the reactor.

(2) In Embodiment 2, the apparatus of the above Embodiment 1 comprises a plurality of reactors comprising N reactors (wherein N is an integer of 2 or higher) from first stage to Nth stage, which reactors are connected in series on the basis of the transportation direction of the liquid phase containing the reaction products, wherein at least one reactor among the reactors of the first stage to the (N-1)th stage is the reactor recited in claim 1, and wherein the apparatus has a means for taking out the liquid phase from the reactor, and a means for supplying the liquid phase to the subsequent reactor.

(3) In Embodiment 3, the apparatus of the above Embodiment 2 comprises a means for taking out the liquid phase containing the reaction product from each reactor, and a means for supplying the liquid phase to the subsequent reactor, provided that each reactor has a subsequent reactor.

(4) In Embodiment 4, the apparatus of any of above Embodiments 1-3, comprising a means for independently

supplying the oxygen-containing, the olefin and hydrogen to the reactor.

(5) In Embodiment 5, the apparatus of any of above Embodiments 1-3 comprises a means for independently supplying an oxygen/olefin-containing gas containing the oxygen-containing gas and the olefin and hydrogen to the reactor.

(6) In Embodiment 6, the apparatus of any of above Embodiments 1-3 comprises a means for supplying an oxygen/olefin/hydrogen-containing gas containing the oxygen-containing gas, the olefin and hydrogen to the reactor.

(7) In Embodiment 7, the apparatus of above Embodiment 5 further comprises a preparation apparatus for preparing an oxygen/olefin-containing gas, which preparing apparatus comprising a first mixer for preparing an oxygen/steam/olefin-containing gas by mixing an oxygen/steam-containing gas containing oxygen and steam with an olefin; and a condenser for preparing an oxygen/steam/olefin-containing gas with a decreased amount of steam, as an oxygen/olefin-containing gas, by removing steam from the oxygen/steam/olefin-containing gas by condensation.

(8) In Embodiment 8, the apparatus of above Embodiment 5 further comprises a preparation apparatus for preparing an oxygen/olefin/hydrogen-containing gas, which preparation apparatus comprising a first mixer for preparing an oxygen/steam/olefin-containing gas by mixing an oxygen/steam-containing gas containing oxygen and steam with an olefin; a condenser for preparing an oxygen/steam/olefin-containing gas with a decreased amount of steam, as an oxygen/olefin-containing gas, by removing steam from the oxygen/steam/olefin-containing gas by condensation; and a second mixer for preparing an oxygen/steam/olefin/hydrogen-containing gas, as an oxygen/olefin/hydrogen-containing gas, by mixing the oxygen/steam/olefin-containing gas with a decreased amount of steam with hydrogen.

(9) In Embodiment 9, the apparatus of any of Embodiments 1-8 comprises a means for mixing the vapor phase and/or liquid phase therein.

(10) In Embodiment 10, in the apparatus of Embodiment 9, the means for mixing is a compressor for supplying the vapor phase in the reactor to the liquid phase.

(11) In Embodiment 11, in the apparatus of Embodiment 9, the means for mixing is an ejector for externally circulating the liquid phase in the reactor.

(12) In Embodiment 12, in the apparatus of Embodiment 9, the means for mixing is a stirrer for mixing the vapor phase in the reactor with the liquid phase.

(13) In Embodiment 13, in the apparatus of Embodiment 12, the stirrer comprises a self-suction type gas-liquid contact stirrer.

(14) In Embodiment 14, a method for producing an epoxy compound derived from an olefin by causing reaction of hydrogen, oxygen and the olefin in a liquid solvent in the presence of a catalyst in a reactor is **characterized in that** the process comprises a step of continuously supplying hydrogen, an oxygen-containing gas with an oxygen concentration of at least 90% by volume and the olefin to the vapor phase and/or liquid phase of the reactor; a step of reacting hydrogen, oxygen and the olefin in the liquid phase; and a step of taking out the liquid phase containing the reaction product from the reactor.

(15) In Embodiment 15, in the method of Embodiment 14, the vapor phase of the reactor contains an inert gas and the liquid phase of the reactor contains the liquid solvent and the catalyst.

(16) In Embodiment 16, in the method of any of Embodiments 14 and 15, the oxygen concentration in the vapor phase in the reactor is adjusted to outside the explosion range.

(17) In Embodiment 17, in the method of Embodiment 16, the oxygen concentration in the vapor phase in the reactor is adjusted equal to 5% or less.

(18) In Embodiment 18, in the method of any of Embodiments 14 and 15, the total amount of the inert gas being supplied to the reactor is adjusted equal to 10% by volume or less in the total amount of hydrogen, the oxygen-containing gas and the olefin by supplying hydrogen, the oxygen-containing gas and the olefin, wherein at least one of hydrogen, the oxygen-containing gas and the olefin further contains the inert gas.

(19) In Embodiment 19, the method of any of Embodiments 14-18 comprises using a plurality of reactors comprising N reactors (wherein N is an integer of 2 or higher) from first stage to Nth stage, which reactors are connected in series on the basis of the transportation direction of the liquid phase containing the reaction products; taking out a liquid phase containing reaction products from at least one reactor among reactors of the first stage to the (N-1)th stage and supplying the liquid phase to the subsequent reactor; and supplying hydrogen, oxygen and an olefin to the vapor phase and/or liquid phase of at least one reactor, wherein the vapor phase of at least one reactor contains an inert gas.

(20) In Embodiment 20, the method of Embodiment 19 comprises taking out the liquid phase containing reaction products from the reactor of the Nth stage.

(21) In Embodiment 21, in the method of Embodiment 19 or 20, hydrogen and oxygen are not supplied to the reactor of the Nth stage.

(22) In Embodiment 22, the method of Embodiment 20 or 21 comprises taking out the liquid phase containing the reaction products from each of the reactors and supplying the liquid phase to the subsequent reactor, provided that each reactor has a subsequent reactor.

(23) In Embodiment 23, in the method of any of Embodiments 14-22, the inert gas contained in the vapor phase in

the reactor is at least one gas selected from the group consisting of steam, nitrogen, argon, carbon dioxide, helium, methane, ethane and propane.

(24) In Embodiment 24, the method of any of Embodiments 14-23 comprises independently supplying oxygen-containing gas, olefin and hydrogen to the reactor.

(25) In Embodiment 25, the method of any of Embodiments 14-23 comprises independently supplying oxygen/olefin-containing gas containing the oxygen-containing gas and the olefin and hydrogen to the reactor.

(26) In Embodiment 26, the method of any of Embodiments 14-23 comprises supplying an oxygen/olefin/hydrogen-containing gas containing the oxygen-containing gas, the olefin and hydrogen to the reactor.

(27) In Embodiment 27, the method of Embodiment 25 further comprises preparing the oxygen/olefin-containing gas beforehand, wherein the preparing comprising a first mixing step of preparing an oxygen/steam/olefin-containing gas by mixing the oxygen/steam-containing gas containing oxygen and steam with an olefin; and a steam removal step of preparing an oxygen/steam/olefin-containing gas with a decreased amount of steam, as an oxygen/olefin-containing gas, by removing steam from the oxygen/steam/olefin-containing gas by condensation.

(28) In Embodiment 28, the method of Embodiment 26 further comprises preparing the oxygen/olefin/hydrogen-containing gas beforehand, wherein the preparing comprising a first mixing step of preparing an oxygen/steam/olefin-containing gas by mixing the oxygen/steam-containing gas containing oxygen and steam with an olefin; a steam removal step of preparing an oxygen/steam/olefin-containing gas with a decreased amount of steam, as an oxygen/olefin-containing gas, by removing steam from the oxygen/steam/olefin-containing gas by condensation; and a second mixing step of preparing an oxygen/steam/olefin/hydrogen-containing gas as the oxygen/olefin/hydrogen-containing gas by mixing the oxygen/steam/olefin-containing gas with a decreased amount of steam with hydrogen.

(29) In Embodiment 29, in the method of any of Embodiments 14-28, the liquid solvent comprises water.

(30) In Embodiment 30, in the method of any of Embodiments 14-29, the oxygen-containing gas having purity of at least 90% by volume is pure oxygen, or the oxygen-containing gas that obtained by the oxygen obtained by cryogenic separation or pressure swing adsorption method.

(31) In Embodiment 31, in the method of any of Embodiments 14-30, the olefin is propylene.

(32) In Embodiment 32, in the method of any of Embodiments 14-31, the liquid phase contains a quinoide compound or a dihydro-form of the quinoid compound.

(33) In Embodiment 33, in the method of any of Embodiments 14-32, the catalyst comprises titanosilicate and noble metal.

(34) In Embodiment 34, in the method of any of Embodiments 14-33, the titanosilicate is any of crystalline titanosilicates having MWW structure and layered titanosilicates

(35) In Embodiment 35, in the method of any of Embodiments 14-34, the noble metal is palladium.

(36) In Embodiment 36, in the method of any of Embodiments 14-35, the liquid solvent comprises acetonitrile.

(37) In Embodiment 37, in the method of any of Embodiments 14-35, the vapor phase and/or liquid phase of the reactor is circulated.

## Claims

1. An apparatus for producing an epoxy compound derived from an olefin by causing a reaction of hydrogen, oxygen, and the olefin in a liquid solvent in the presence of a catalyst in a reactor, wherein the apparatus has a means for continuously supplying an inert gas to the vapor phase and/or liquid phase of the reactor, and a means for taking out the liquid phase containing a reaction product from the reactor, and wherein the reactor has a means for supplying hydrogen, an oxygen-containing gas with an oxygen concentration of at least 90% by volume and the olefin to the vapor phase and/or liquid phase in the reactor.

2. The apparatus of claim 1, comprising a plurality of reactors comprising N reactors (wherein N is an integer of 2 or higher) from first stage to Nth stage, which reactors are connected in series on the basis of the transportation direction of the liquid phase containing the reaction products, wherein at least one reactor among the reactors of the first stage to the (N-1) th stage is the reactor recited in claim 1, and wherein the apparatus has a means for taking out the liquid phase from the reactor, and a means for supplying the liquid phase to the subsequent reactor.

3. The apparatus of claim 2, comprising a means for taking out the liquid phase containing the reaction product from each reactor, and a means for supplying the liquid phase to the subsequent reactor, provided that each reactor has a subsequent reactor.

4. The apparatus of any of claims 1-3, comprising a means for independently supplying the oxygen-containing, the

olefin and hydrogen to the reactor.

**5.** The apparatus of any of claims 1-3, comprising a means for independently supplying an oxygen/olefin-containing gas containing the oxygen-containing gas and the olefin and hydrogen to the reactor.

**6.** The apparatus of any of claims 1-3, comprising a means for supplying an oxygen/olefin/hydrogen-containing gas containing the oxygen-containing gas, the olefin and hydrogen to the reactor.

**7.** The apparatus of claim 5 further comprising a preparation apparatus for preparing an oxygen/olefin-containing gas, which preparing apparatus comprising a first mixer for preparing an oxygen/steam/olefin-containing gas by mixing an oxygen/steam-containing gas containing oxygen and steam with an olefin; and a condenser for preparing an oxygen/steam/olefin-containing gas with a decreased amount of steam, as an oxygen/olefin-containing gas, by removing steam from the oxygen/steam/olefin-containing gas by condensation.

**8.** The apparatus of claim 6 further comprising a preparation apparatus for preparing an oxygen/olefin/hydrogen-containing gas, which preparation apparatus comprising a first mixer for preparing an oxygen/steam/olefin-containing gas by mixing an oxygen/steam-containing gas containing oxygen and steam with an olefin; a condenser for preparing an oxygen/steam/olefin-containing gas with a decreased amount of steam, as an oxygen/olefin-containing gas, by removing steam from the oxygen/steam/olefin-containing gas by condensation; and a second mixer for preparing an oxygen/steam/olefin/hydrogen-containing gas, as an oxygen/olefin/hydrogen-containing gas, by mixing the oxygen/steam/olefin-containing gas with a decreased amount of steam with hydrogen.

**9.** The apparatus of any of claims 1-8, comprising a means for mixing the vapor phase and/or liquid phase therein.

**10.** The apparatus of claim 9, wherein the means for mixing is a compressor for supplying the vapor phase in the reactor to the liquid phase.

**11.** The apparatus of claim 9, wherein the means for mixing is an ejector for externally circulating the liquid phase in the reactor.

**12.** The apparatus of claim 9, wherein the means for mixing is a stirrer for mixing the vapor phase in the reactor with the liquid phase.

**13.** The apparatus of claim 12, wherein the stirrer comprises a self-aspirating type gas-liquid contact stirrer.

**14.** A method for producing an epoxy compound derived from an olefin by causing reaction of hydrogen, oxygen and the olefin in a liquid solvent in the presence of a catalyst in a reactor, **characterized in that** the process comprises a step of continuously supplying hydrogen, an oxygen-containing gas with an oxygen concentration of at least 90% by volume and the olefin to the vapor phase and/or liquid phase of the reactor; a step of reacting hydrogen, oxygen and the olefin in the liquid phase; and a step of taking out the liquid phase containing the reaction product from the reactor.

**15.** The method of claim 14, wherein the vapor phase of the reactor contains an inert gas and the liquid phase of the reactor contains the liquid solvent and the catalyst.

**16.** The method of claim 14 or 15, wherein the oxygen concentration in the vapor phase in the reactor is adjusted to outside the explosion range.

**17.** The method of claim 16, wherein the oxygen concentration in the vapor phase in the reactor is adjusted equal to 5% or less.

**18.** The method of claim 14 or 15, wherein the total amount of the inert gas being supplied to the reactor is adjusted equal to 10% by volume or less in the total amount of hydrogen, the oxygen-containing gas and the olefin by supplying hydrogen, the oxygen-containing gas and the olefin, wherein at least one of hydrogen, the oxygen-containing gas and the olefin further contains the inert gas.

**19.** The method of any of claims 14-18, comprising using a plurality of reactors comprising N reactors (wherein N is an integer of 2 or higher) from first stage to Nth stage, which reactors are connected in series on the basis of the

transportation direction of the liquid phase containing the reaction products; taking out a liquid phase containing reaction products from at least one reactor among reactors of the first stage to the (N-1)th stage and supplying the liquid phase to the subsequent reactor; and supplying hydrogen, oxygen and an olefin to the vapor phase and/or liquid phase of at least one reactor, wherein the vapor phase of at least one reactor contains an inert gas.

**20.** The method of claim 19, comprising taking out the liquid phase containing reaction products from the reactor of the Nth stage.

**21.** The method of claims 19 or 20, wherein hydrogen and oxygen are not supplied to the reactor of the Nth stage.

**22.** The method of claims 20 or 21, comprising taking out the liquid phase containing the reaction products from each of the reactors and supplying the liquid phase to the subsequent reactor, provided that each reactor has a subsequent reactor.

**23.** The method of any of claims 14-22, wherein the inert gas contained in the vapor phase in the reactor is at least one gas selected from the group consisting of steam, nitrogen, argon, carbon dioxide, helium, methane, ethane and propane.

**24.** The method of any of claims 14-23, comprising independently supplying oxygen-containing gas, olefin and hydrogen to the reactor.

**25.** The method of any of claims 14-23, comprising independently supplying oxygen/olefin-containing gas containing the oxygen-containing gas and the olefin and hydrogen to the reactor.

**26.** The method of any of claims 14-23, comprising supplying an oxygen/olefin/hydrogen-containing gas containing the oxygen-containing gas, the olefin and hydrogen to the reactor.

**27.** The method of claim 25 further comprising preparing the oxygen/olefin-containing gas beforehand, wherein the preparing comprising a first mixing step of preparing an oxygen/steam/olefin-containing gas by mixing the oxygen/steam-containing gas containing oxygen and steam with an olefin; and a steam removal step of preparing an oxygen/steam/olefin-containing gas with a decreased amount of steam, as an oxygen/olefin-containing gas, by removing steam from the oxygen/steam/olefin-containing gas by condensation.

**28.** The method of claim 26 further comprising preparing the oxygen/olefin/hydrogen-containing gas beforehand, wherein the preparing comprising a first mixing step of preparing an oxygen/steam/olefin-containing gas by mixing the oxygen/steam-containing gas containing oxygen and steam with an olefin; a steam removal step of preparing an oxygen/steam/olefin-containing gas with a decreased amount of steam, as an oxygen/olefin-containing gas, by removing steam from the oxygen/steam/olefin-containing gas by condensation; and a second mixing step of preparing an oxygen/steam/olefin/hydrogen-containing gas as the oxygen/olefin/hydrogen-containing gas by mixing the oxygen/steam/olefin-containing gas with a decreased amount of steam with hydrogen.

**29.** The method of any of claims 14-28, wherein the liquid solvent comprises water.

**30.** The method of any of claims 14-29, wherein the oxygen-containing gas having purity of at least 90% by volume is pure oxygen, or the oxygen-containing gas that obtained by the oxygen obtained by cryogenic separation or pressure swing adsorption method.

**31.** The method of any of claims 14-30, wherein the olefin is propylene.

**32.** The method of any of claims 14-31, wherein the liquid phase contains a quinoide compound or a dihydro-form of the quinoid compound.

**33.** The method of any of claims 14-32, wherein the catalyst comprises titanosilicate and noble metal.

**34.** The method of any of claims 14-33, wherein the titanosilicate is any of crystalline titanosilicates having MWW structure and layered titanosilicates

**35.** The method of any of claims 14-34, wherein the noble metal is palladium.

**36.** The method of any of claims 14-35, wherein the liquid solvent comprises acetonitrile.

**37.** The method of any of claims 14-35, wherein the vapor phase and/or liquid phase of the reactor is circulated.

[Fig. 1]

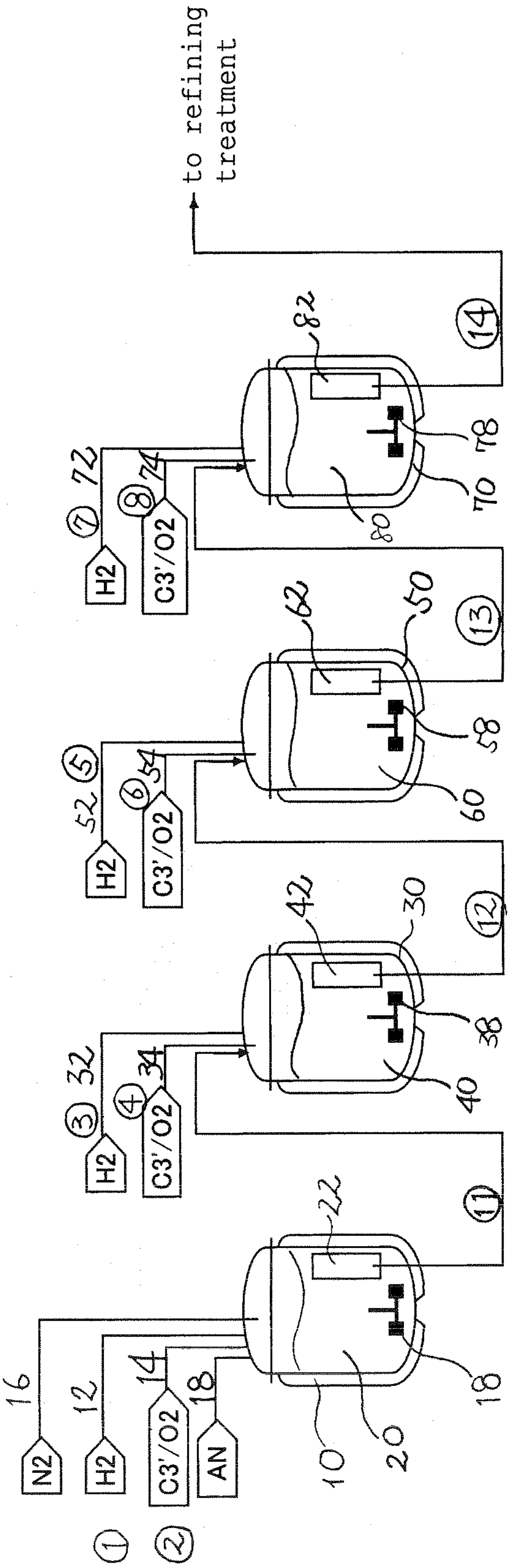
to refining treatment
N2
H2
C3'/O2
AN
16
12
14
18
10
20
22
H2
C3'/O2
32
34
30
38
40
42
52
54
50
58
60
62
72
74
70
78
80
82
1
2
3
4
5
6
7
8
11
12
13
14

[Fig. 2]

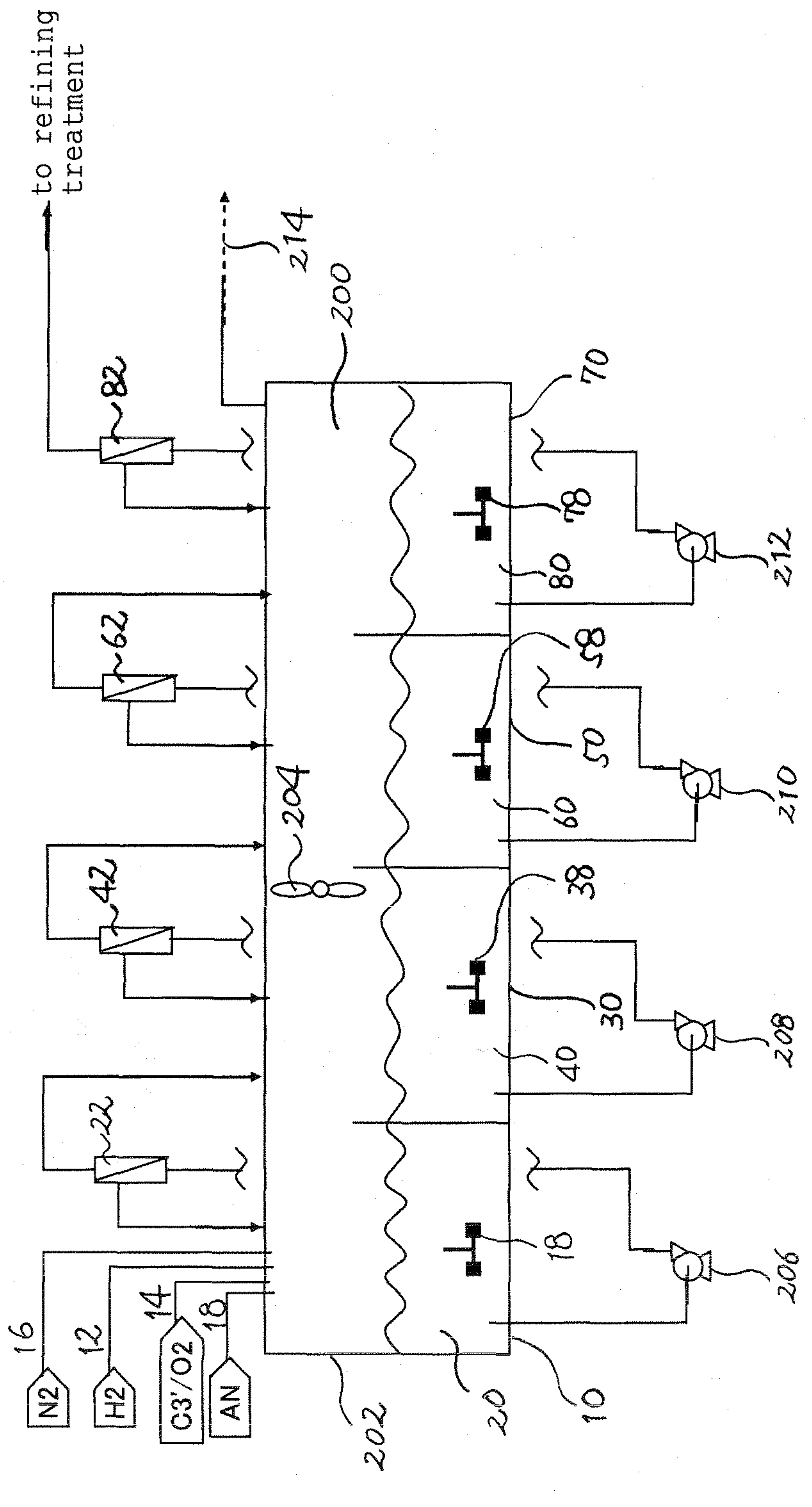
to refining treatment
214
200
82
70
78
80
212
62
58
50
60
210
204
42
38
30
40
208
22
18
206
16
12
14
18
N2
H2
C3'/O2
AN
202
20
10

[Fig. 3]

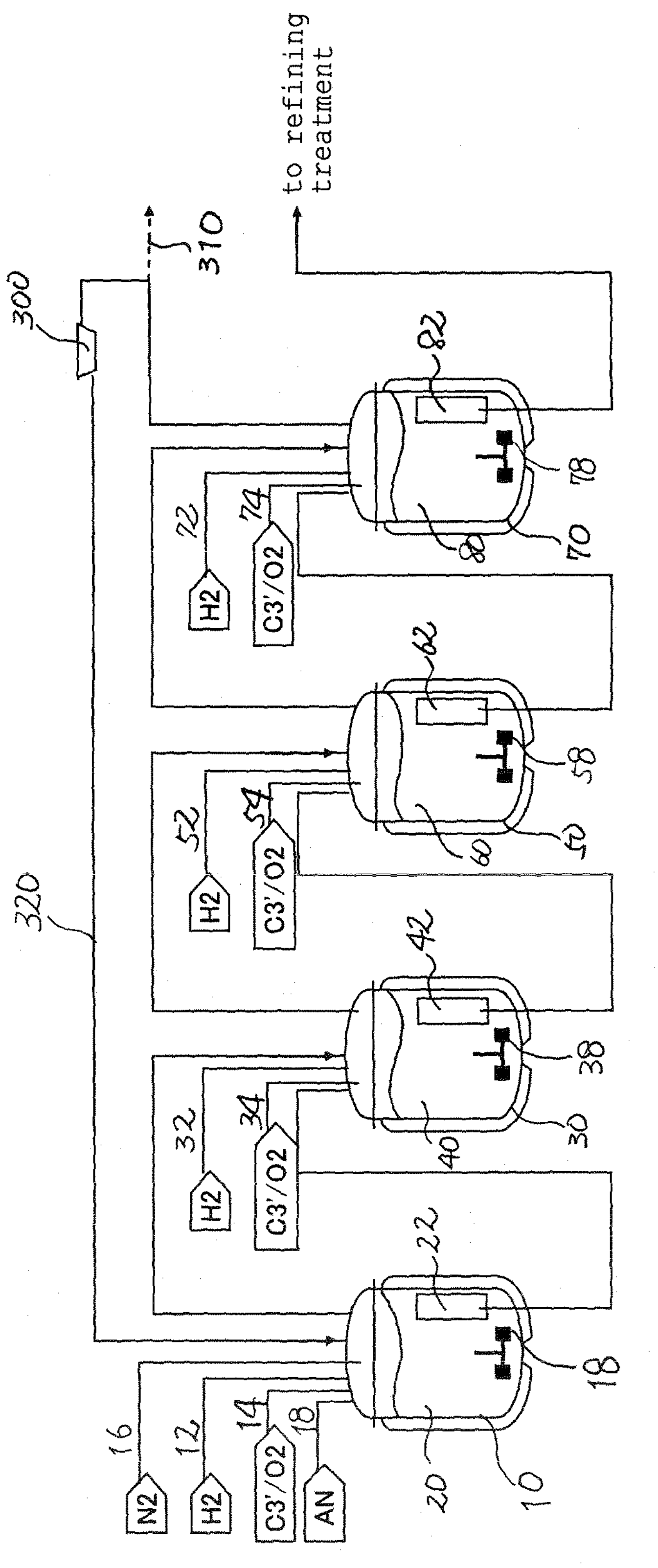
to refining treatment
310
300
320
N2
H2
C3'/O2
AN
16
12
14
18
10
20
22
18
32
34
30
38
40
42
52
54
50
58
60
62
72
74
70
78
80
82

[Fig. 4]

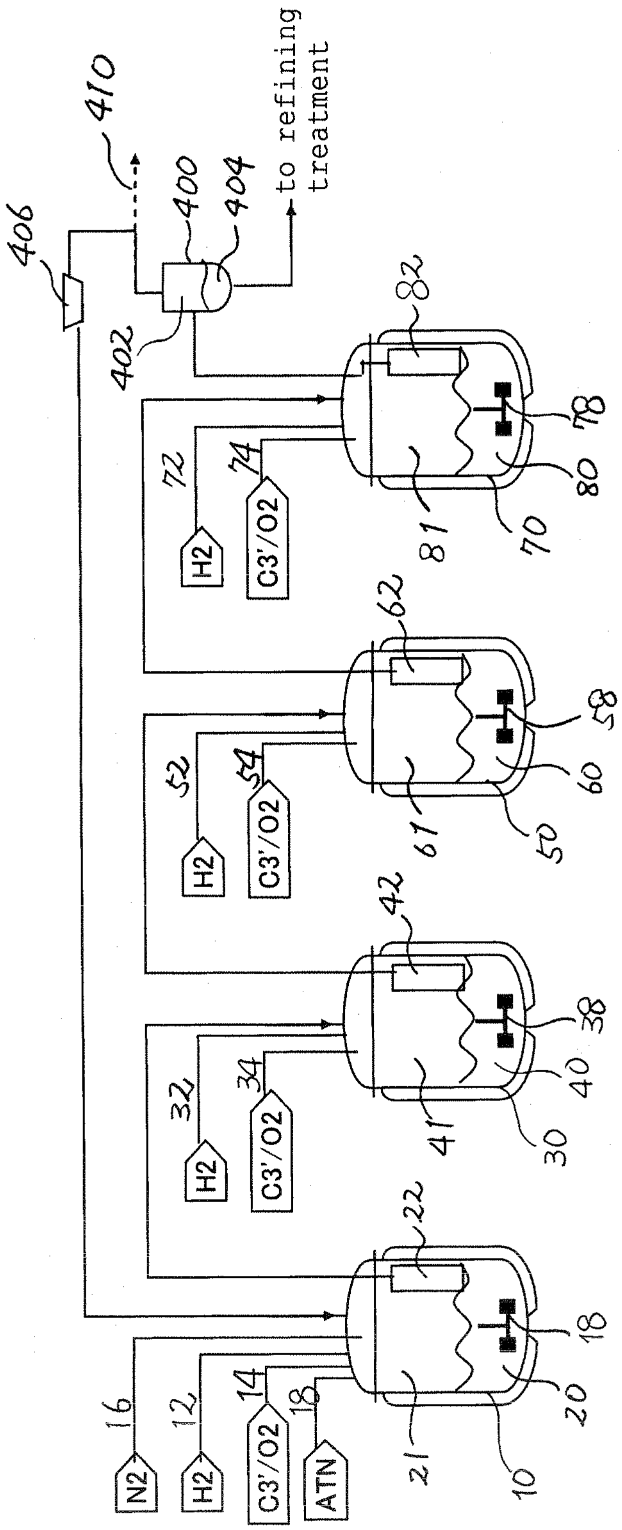
to refining treatment
410
406
400
404
402
82
72
74
H2
C3'/O2
81
70
80
78
62
52
54
H2
C3'/O2
61
50
60
58
42
32
34
H2
C3'/O2
41
30
40
38
22
16
12
14
18
N2
H2
C3'/O2
ATN
21
10
20
18

[Fig. 5]

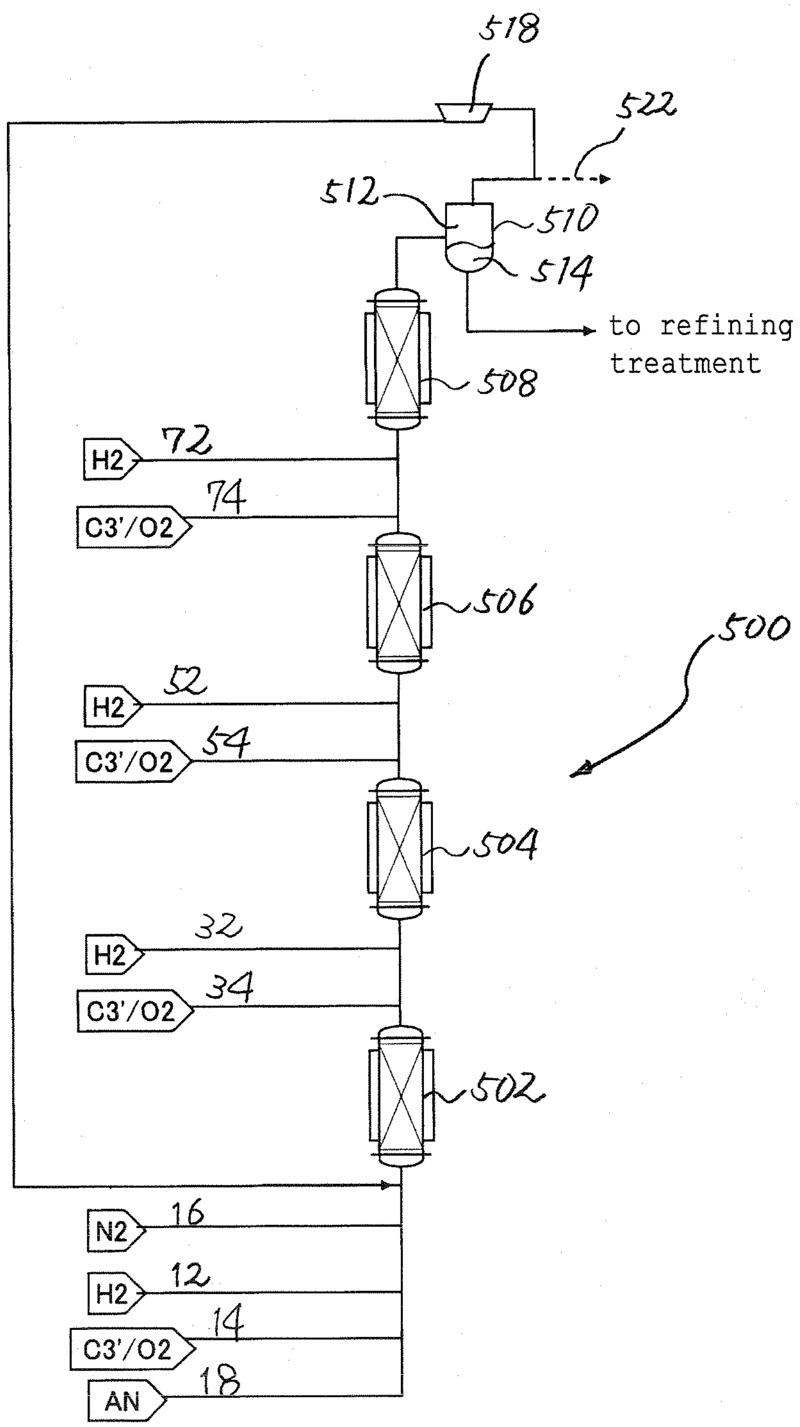
518
522
512
510
514
to refining treatment
508
H2
72
C3'/O2
74
506
500
H2
52
C3'/O2
54
504
H2
32
C3'/O2
34
502
N2
16
H2
12
C3'/O2
14
AN
18

[Fig. 6]

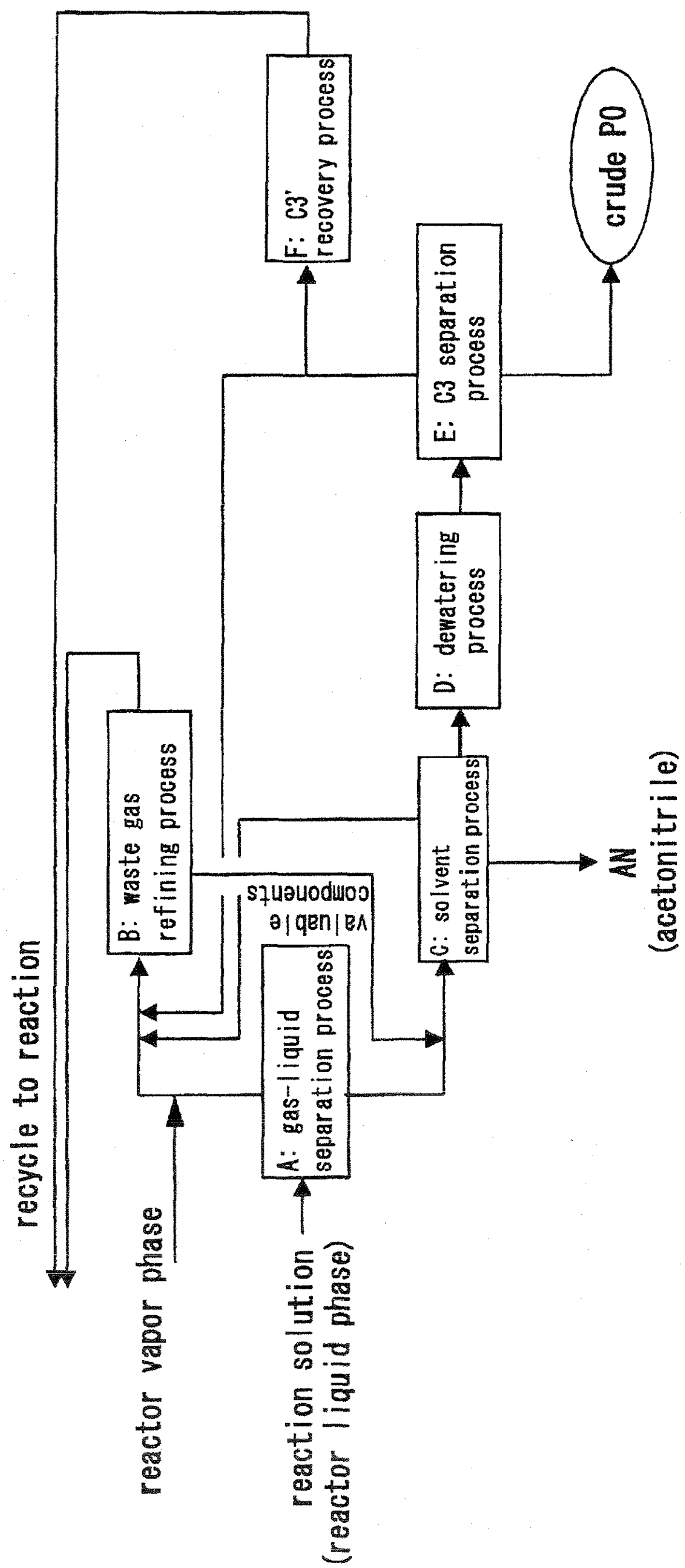
recycle to reaction
reactor vapor phase
reaction solution (reactor liquid phase)
A: gas-liquid separation process
B: waste gas refining process
valuable components
C: solvent separation process
AN (acetonitrile)
D: dewatering process
E: C3 separation process
F: C3' recovery process
crude PO

[Fig. 7]

Table 3

| | N2 | ① | ② | ③ | ④ | ⑤ | ⑥ | ⑦ | ⑧ |
|---|---|---|---|---|---|---|---|---|---|
| | mol/hr | mol/hr | mol/hr | mol/hr | mol/hr | mol/hr | mol/hr | mol/hr | mol/hr |
| C3' | 0.00E+00 | 0.00E+00 | 1.05E-02 | 0.00E+00 | 5.14E-03 | 0.00E+00 | 5.14E-03 | 0.00E+00 | 5.14E-03 |
| O2 | 0.00E+00 | 0.00E+00 | 7.74E-03 | 0.00E+00 | 7.27E-03 | 0.00E+00 | 7.27E-03 | 0.00E+00 | 7.27E-03 |
| H2 | 0.00E+00 | 1.11E-02 | 0.00E+00 | 1.01E-02 | 0.00E+00 | 1.01E-02 | 0.00E+00 | 1.01E-02 | 0.00E+00 |
| N2 | 1.45E-02 | 0.00E+00 | 0.00E+00 | 0.00E+00 | 0.00E+00 | 0.00E+00 | 0.00E+00 | 0.00E+00 | 0.00E+00 |
| PO | 0.00E+00 | 0.00E+00 | 0.00E+00 | 0.00E+00 | 0.00E+00 | 0.00E+00 | 0.00E+00 | 0.00E+00 | 0.00E+00 |
| AN | 0.00E+00 | 0.00E+00 | 0.00E+00 | 0.00E+00 | 0.00E+00 | 0.00E+00 | 0.00E+00 | 0.00E+00 | 0.00E+00 |
| H2O | 0.00E+00 | 0.00E+00 | 0.00E+00 | 0.00E+00 | 0.00E+00 | 0.00E+00 | 0.00E+00 | 0.00E+00 | 0.00E+00 |
| C3 | 0.00E+00 | 0.00E+00 | 0.00E+00 | 0.00E+00 | 0.00E+00 | 0.00E+00 | 0.00E+00 | 0.00E+00 | 0.00E+00 |

| | AN | ⑪ | ⑫ | ⑬ | ⑭ |
|---|---|---|---|---|---|
| | mol/hr | mol/hr | mol/hr | mol/hr | mol/hr |
| C3' | 0.00E+00 | 5.40E-03 | 5.40E-03 | 5.40E-03 | 5.40E-03 |
| O2 | 0.00E+00 | 4.66E-04 | 4.66E-04 | 4.66E-04 | 4.66E-04 |
| H2 | 0.00E+00 | 1.10E-03 | 1.10E-03 | 1.10E-03 | 1.10E-03 |
| N2 | 0.00E+00 | 1.45E-02 | 1.45E-02 | 1.45E-02 | 1.45E-02 |
| PO | 0.00E+00 | 4.62E-03 | 9.25E-03 | 1.39E-02 | 1.85E-02 |
| AN | 1.72E+00 | 1.72E+00 | 1.72E+00 | 1.72E+00 | 1.72E+00 |
| H2O | 9.82E-01 | 9.91E-01 | 1.00E+00 | 1.01E+00 | 1.02E+00 |
| C3 | 0.00E+00 | 5.37E-05 | 1.07E-04 | 1.61E-04 | 2.15E-04 |

[Fig. 8]

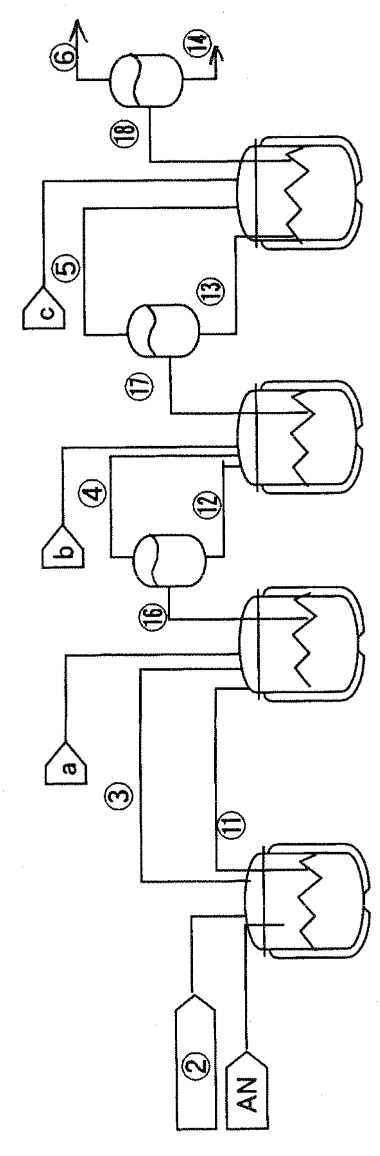

[Fig. 9]

Table 4

| | ② | ③ | ④ | ⑤ | ⑥ | a | b | c | d |
|---|---|---|---|---|---|---|---|---|---|
| | mol/hr | mol/hr | mol/hr | mol/hr | mol/hr | mol/hr | mol/hr | mol/hr | mol/hr |
| C3' | 3.70E−02 | 2.65E−02 | 2.60E−02 | 2.54E−02 | 2.47E−02 | 4.09E−03 | 4.09E−03 | 4.09E−03 | 4.09E−03 |
| O2 | 3.19E−02 | 2.42E−02 | 2.05E−02 | 1.69E−02 | 1.32E−02 | 3.52E−03 | 3.52E−03 | 3.52E−03 | 3.52E−03 |
| H2 | 9.10E−02 | 7.99E−02 | 7.99E−02 | 8.00E−02 | 8.01E−02 | 1.01E−02 | 1.01E−02 | 1.01E−02 | 1.01E−02 |
| N2 | 7.07E−01 | 6.92E−01 | 7.70E−01 | 8.48E−01 | 9.26E−01 | 7.81E−02 | 7.81E−02 | 7.81E−02 | 7.81E−02 |
| PO | 0.00E+00 | 6.20E−04 | 1.33E−03 | 2.14E−03 | 3.03E−03 | 0.00E+00 | 0.00E+00 | 0.00E+00 | 0.00E+00 |
| AN | 0.00E+00 | 4.27E−02 | 4.65E−02 | 5.02E−02 | 5.40E−02 | 0.00E+00 | 0.00E+00 | 0.00E+00 | 0.00E+00 |
| H2O | 0.00E+00 | 1.83E−02 | 2.00E−02 | 2.17E−02 | 2.34E−02 | 0.00E+00 | 0.00E+00 | 0.00E+00 | 0.00E+00 |
| C3 | 0.00E+00 | 4.60E−04 | 9.28E−04 | 1.40E−03 | 1.88E−03 | 0.00E+00 | 0.00E+00 | 0.00E+00 | 0.00E+00 |
| Total | 8.67E−01 | 8.85E−01 | 9.65E−01 | 1.05E+00 | 1.13E+00 | 9.57E−02 | 9.57E−02 | 9.57E−02 | 9.57E−02 |

| | AN | ⑪ | ⑫ | ⑬ | ⑭ | ⑯ | ⑰ | ⑱ |
|---|---|---|---|---|---|---|---|---|
| | mol/hr | mol/hr | mol/hr | mol/hr | mol/hr | mol/hr | mol/hr | mol/hr |
| C3' | 0.00E+00 | 5.40E−03 | 4.86E−03 | 4.39E−03 | 3.98E−03 | 3.08E−02 | 2.98E−02 | 2.87E−02 |
| O2 | 0.00E+00 | 4.66E−04 | 3.63E−04 | 2.75E−04 | 2.00E−04 | 2.09E−02 | 1.71E−02 | 1.34E−02 |
| H2 | 0.00E+00 | 1.10E−03 | 1.01E−03 | 9.30E−04 | 8.64E−04 | 8.09E−02 | 8.09E−02 | 8.09E−02 |
| N2 | 0.00E+00 | 1.45E−02 | 1.48E−02 | 1.50E−02 | 1.52E−02 | 7.85E−01 | 8.63E−01 | 9.41E−01 |
| PO | 0.00E+00 | 4.00E−03 | 7.91E−03 | 1.17E−02 | 1.55E−02 | 9.25E−03 | 1.39E−02 | 1.85E−02 |
| AN | 1.72E+00 | 1.68E+00 | 1.68E+00 | 1.67E+00 | 1.67E+00 | 1.72E+00 | 1.72E+00 | 1.72E+00 |
| H2O | 9.82E−01 | 9.73E−01 | 9.80E−01 | 9.87E−01 | 9.95E−01 | 1.00E+00 | 1.01E+00 | 1.02E+00 |
| C3 | 0.00E+00 | 5.37E−05 | 9.94E−05 | 1.39E−04 | 1.73E−04 | 1.03E−03 | 1.54E−03 | 2.06E−03 |
| Total | 2.71E+00 | 2.68E+00 | 2.69E+00 | 2.69E+00 | 2.70E+00 | 3.65E+00 | 3.74E+00 | 3.83E+00 |

[Fig. 10]

Table 5

| | second reactor | | third reactor | | fourth reactor | |
|---|---|---|---|---|---|---|
| | composition | partial pressure | composition | partial pressure | composition | partial pressure |
| | mol% | kPa | mol% | kPa | mol% | kPa |
| C3' | 2.7 | 24.2 | 2.4 | 21.8 | 2.2 | 19.8 |
| O2 | 2.1 | 19.1 | 1.6 | 14.5 | 1.2 | 10.5 |
| H2 | 8.3 | 74.5 | 7.7 | 68.9 | 7.1 | 64.0 |
| N2 | 79.8 | 718.0 | 81.1 | 729.8 | 82.2 | 739.9 |
| PO | 0.1 | 1.2 | 0.2 | 1.8 | 0.3 | 2.4 |
| AN | 4.8 | 43.3 | 4.8 | 43.2 | 4.8 | 43.1 |
| H2O | 2.1 | 18.7 | 2.1 | 18.7 | 2.1 | 18.7 |
| C3 | 0.1 | 0.9 | 0.1 | 1.2 | 0.2 | 1.5 |
| Total | 100.0 | 900 | 100.0 | 900 | 100.0 | 900 |

[Fig. 11]

**Table 6**

| residence time (hour) | first reactor | second reactor | third reactor | fourth reactor |
|---|---|---|---|---|
| | hr | hr | hr | hr |
| | 1.5 | 1.6 | 1.8 | 1.9 |

**INTERNATIONAL SEARCH REPORT**

International application No.
PCT/JP2008/062326

A. CLASSIFICATION OF SUBJECT MATTER
*C07D301/06*(2006.01)i, *B01J29/89*(2006.01)i, *C07D301/08*(2006.01)i, *C07D303/04*(2006.01)i, *C07B61/00*(2006.01)n

According to International Patent Classification (IPC) or to both national classification and IPC

B. FIELDS SEARCHED

Minimum documentation searched (classification system followed by classification symbols)
C07D301/06, B01J29/89, C07D301/08, C07D303/04, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2004-506627 A (POLIMERI EUROPA S.P.A.), 04 March, 2004 (04.03.04), & WO 2002/014299 A1 | 1-13,37 |
| X | JP 4-352771 A (Tosoh Corp.), 07 December, 1992 (07.12.92), (Family: none) | 14-18,23-25, 29-31,33,35 |
| X<br>Y | JP 2004-269380 A (Sumitomo Chemical Co., Ltd.), 30 September, 2004 (30.09.04), (Family: none) | 19-26,29,34, 36<br>32 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 October, 2008 (01.10.08) | 14 October, 2008 (14.10.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/062326

C (Continuation). DOCUMENTS CONSIDERED TO BE RELEVANT

| Category* | Citation of document, with indication, where appropriate, of the relevant passages | Relevant to claim No. |
|---|---|---|
| Y | JP 10-72455 A (Sumitomo Chemical Co., Ltd.), 17 March, 1998 (17.03.98), & US 5840934 A & EP 812836 A1 & DE 69732739 D & DE 69732739 T & CA 2207727 A & SG 74015 A & CA 2207727 A1 | 32 |
| A | JP 2005-514364 A (ARCO CHEMICAL TECHNOLOGY, L.P.), 19 May, 2005 (19.05.05), & US 6399794 B1 & WO 2003/044001 A1 & DE 60208433 D & DE 60208433 T & CA 2465783 A & BR 214109 A & AT 314355 T & ES 2250746 T & CN 1585759 A | 1-37 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003510314 A
- JP 2005514364 A
- JP 2003032745 A

### Non-patent literature cited in the description

- *Zeolites,* 1995, vol. 15, 236-242
- *Journal of Catalysis,* 2001, vol. 199, 41-47
- *Chemistry. Letters,* 2000, 774-775
- *Zeolites,* 1995, vol. 15, 519-525
- *Journal of Catalysis,* 1991, vol. 130, 1-8
- *Angewante Chemie International Edition,* 2004, vol. 43, 236-240
- *Microporous Material,* 1997, vol. 10, 259-271
- *Chemical Communications,* 1996, 145-146
- *Chemistry of Materials,* 2002, vol. 14, 1657-1664
- *Microporous and Mesoporous Materials,* 2002, vol. 52, 11-18
- **HASHIMOTO Kenji.** reaction Engineering. Baifukan, 10 October 1995, 54